# EUROPEAN PATENT SPECIFICATION

(11) **EP 1 221 937 B1**
(45) Date of publication and mention of the grant of the patent: **15.12.2004**
(21) Application number: 00968669.2
(22) Date of filing: 04.10.2000
(51) Int. Cl.: A61K 9/00, A61L 27/24, A61L 27/38, A61L 27/44, A61L 27/54, C07K 14/61, A61K 48/00

(54) **HYBRID MATRICES AND HYBRID MATRIX MIXTURES**
HYBRIDE MATRIZEN UND GEMISCHE DAVON
MATRICES HYBRIDES ET MELANGES DE MATRICES HYBRIDES

(30) Priority: 05.10.1999 US 413715; 14.09.2000 US 662037
(43) Date of publication of application: 17.07.2002
(73) Proprietor: TRANSKARYOTIC THERAPIES, INC., Cambridge, MA 02139 (US)
(72) Inventor: MINEAU-HANSCHKE, Rochelle, Andover, MA 01810 (US); LAMSA, Justin, Chace, Westminster, MA 01473 (US); ABALOS-COYLE, Deborah, Framingham, MA 01701 (US)
(74) Representative: Ruffles, Graham Keith
(86) International application number: PCT/US2000/027362
(87) International publication number: WO 2001/024842

(56) References cited:
- WO-A-97/15195
- US-A- 5 906 817
- KJELL NILLSON, FERENC BUZSAKY, KLAUS MOSBACH: "Growth of anchorage-dependent cells on macroporous microcarriers" BIO/TECHNOLOGY, vol. 4, 1986, pages 989-990, XP000990412

## Description

The field of the invention is medical devices used *in vivo* or *in vitro* for production and delivery of medically useful substances.

### Background of the Invention

The means used to deliver medically useful substances can significantly affect their efficacy. The standard route of administration for many such substances is either oral, intravenous, or subcutaneous. Each has inherent limitations which can affect the therapeutic utility of the substances being delivered. Furthermore, many protein-based drugs have short half-lives and low bioavailabilities, factors that must be considered in their formulation and delivery. Although various devices have been developed to deliver medically useful substances, including portable pumps and catheters, there is still a significant need for improved delivery devices.

Many medically useful substances, including proteins, glycoproteins, and some peptide and nonpeptide hormones, are more efficiently produced by cultured cells than via artificial synthetic routes. Appropriate cells are typically cultured in bioreactors, and the desired product purified therefrom for administration to the patient by standard means, e.g., orally or by intravenous or subcutaneous injection. Alternatively, the cells can be implanted directly into the patient, where they produce and deliver the desired product (see, e.g., U.S. Patent Nos. 6,063,630 and 6,054,288). While this method has a number of theoretical advantages over injection of the product itself, including the possibility that normal cellular feedback mechanisms may be harnessed to allow the delivery of physiologically appropriate levels of the product, it introduces additional complexities. One of these concerns the appropriate environment for the cells at the time of implantation. It would be desirable to organize the cells of the implant in a form that is compatible with the natural *in vivo* environment of the cell type comprising the implant (fibroblasts, for example, exist naturally in a rich network of extracellular matrix composed primarily of collagen). There is also a need in some cases to ensure that the implanted cells remain localized to a defined site in the patient's body, so that they can be monitored and perhaps removed when no longer needed.

One technique that has been tested for this purpose utilizes an implantation device consisting of a solid, unitary piece of Collagen gel (a "collagen matrix") in which the cells are embedded (e.g. Bell, U.S. Patent No. 4,485,096 and U.S. Patent No. 5,965,125). Other substances, such as polytetrafluoro-ethylne (PTFE) fibers (Moullier et al., Nature Genetics, 4:154, 1993; WO 94/24298), may be included in the collagen implant to impart strength or other desirable characteristics to the collagen gel.

Thus, for example, US 5,906,817 relates an implant obtained by assembling in vitro various elements in order to form a neo-organ which is introduced preferably in the peritoneal cavity of the receipient. The implant comprises a biocompatible support intended to the biological anchoring of cells; cells having the capacity of expressing and secreting naturally or after recombination a predetermined compound, for example a compound having a therapeutical interest; and a constituent capable of inducing and/or promoting the gelling of said cells. It also relates to a kit for the preparation of the implant as well as to a new recombinant retroviral vector comprising a provirus DNA sequence modified in that the genes gag, pol and env have been deleted at least partially so as to obtain a proviral DNA capable of replication. It also relates to recombinant cells comprising the new retroviral vector.
WO 97/15195 provides an implantable device having a body of matrix material made up of insoluble collagen fibrils, and disposed therewithin: (a) a plurality of vertebrate cells; and (b) a plurality of microspheres each of which consists primarily of one or more of the following materials: collagen, polystyrene, dextran, polyacrylamide, cellulose, calcium alginate, latex, polysulfone, or glass.

### Summary of the Invention

According to the present invention, there is provided a composition comprising a body of matrix material comprising insoluble collagen fibrils, there being embedded within the body of matrix material
(a) a population of cultured vertebrate cells genetically engineered to express a polypeptide;
(b) a plurality of microcarriers; and
(c) an agent bound to a solid substrate embedded in the body of the matrix material,
wherein the agent is selected from the group consisting of a factor which promotes vascularization, a cytokine, a growth factor and ascorbic acid.

The present invention further provides a method of making such a composition, comprising
forming a mixture comprising:
(a) a plurality of cultured vertebrate cells genetically engineered to express a polypeptide;
(b) a plurality of microcarriers;
(c) a solution comprising soluble collagen; and
(d) an agent bound to a solid substrate, wherein the agent is selected from the group consisting of a factor that promotes vascularization, a cytokine, a growth factor, and ascorbic acid;
subjecting the soluble collagen in the mixture to conditions effective to form a gel; and
exposing the gel to culture conditions which cause the gel to contract, thereby forming the body of the composition.

The present invention further provides for the use of a population of cultured vertebrate cells genetically engineered to express a polypeptide, in the preparation of a composition of the invention for the treatment of a patient in need thereof wherein the cultured vertebrate cells secrete the polypeptide.

In a related aspect, the present invention provides a mixture comprising, suspended in an aqueous collagen solution,
(a) a plurality of cultured vertebrate cells genetically engineered to express a polypeptide,
(b) a plurality of microcarriers, and
(c) at least one agent selected from the group consisting of a factor which promotes vascularization, a cytokine, a growth factor, and ascorbic acid, wherein the at least one agent is bound to a solid substrate suspended in the collagen solution,
wherein there is sufficient collagen in the mixture to allow the mixture to gel at a pH above 5.

The present invention also provides a vessel comprising, suspended in an aqueous collagen solution,
(a) a population of cultured vertebrate cells genetically engineered to express a polypeptide;
(b) a plurality of microcarriers; and
(c) an agent associated with a solid substrate, wherein the agent is selected from the group consisting of a factor which promotes vascularization, a cytokine, a growth factor, and ascorbic acid,
wherein there is sufficient collagen in the mixture to allow the mixture to gel at a pH above 5.

### Details of the Invention

It has been found that the function of collagen matrices can be substantially improved by the addition of microcarriers (i.e., microspheres of any shape) to the collagen matrix, thereby forming what is herein termed a "hybrid matrix". This can be accomplished by mixing microcarriers with the cells and soluble collagen prior to gelling of the collagen to form the matrix. In order to improve the viability, growth, and/or function of the embedded cells, the matrices of the invention can contain solid substrates (e.g., agarose beads, collagen beads, collagen threads, or collagen or non-collagen fibers) coated with, or encapsulating, one or more of the agents listed below.

Applicants have also discovered that cells can be introduced into the body of an animal by suspending microcarriers and the cells (and optional solid substrates associated with (e.g., bound to, coated with, or encapsulating) one or more of the agents listed below) in a fluid, and introducing the suspension into the body, e.g., by injection. The suspension can include soluble collagen in addition to the cells, microcarriers and solid substrates coated with, or encapsulating, any of the agents described herein. These components are mixed together and delivered into the body of an animal as a liquid suspension. The delivery can be via an injectable system such as a syringe with an attached needle or catheter. Using macroporous microcarriers significantly increases the surface area for cellular attachment, and can also provide protection for cells that attach and migrate into the pores of the microcarriers. In mixtures containing collagen, the collagen portion polymerizes to form a solid mass of collagen gel in the animal's body at the delivery site. This gel forms *in situ* around the suspended components (cells, microcarriers, and optional solid substrates). The gel can help to keep the injected components in a defined space, the dimensions of which will depend on the volume of the implanted material, the spatial dimensions of the implant site, and the structure of the surrounding tissue. The presence of collagen can protect the cells from the shear stress that occurs during the implantation process. In addition, collagen may help decrease the potential destruction of the implanted cells by the host's inflammatory cells by providing a protective barrier. The mixtures with collagen are called "collagen matrix mixtures" (CMM) and the solid matrices formed using the CMM (either *in vivo* or *in vitro*) are termed "collagen matrices" (CM).

If desired, the microcarriers and cells can be cultured together for a period that permits the cells to adhere to the microcarriers before addition of the non-gelled collagen solution and solid substrates; alternatively, the three or four constituents can be mixed essentially simultaneously or in any desired order, followed, if desired, by gelation of the soluble collagen *in vitro*, to form a gelled mixture consisting of insoluble collagen fibrils, cells and microcartiers. The gelled mixture gradually becomes smaller through the exclusion of liquid, to form a solid, relatively resilient, implantable unit that contains both the microcarriers and the cells (and optional solid substrates associated with (e.g., bound to, coated with, or encapsulating) any of the agents listed below) embedded in the insoluble collagen fibril network. When the microcarriers are included, the resulting CMM is herein termed a "hybrid collagen matrix mixture" (HCMM) and a matrix produced from such a HCMM is termed a "hybrid collagen matrix" (HCM). Mixtures without collagen or with a collagen alternative (see below) are termed "matrix mixtures"; when such mixtures contain microcarriers, they are also designated "hybrid matrix mixture"("HMM"). It is understood that the microcarriers can be made of collagen or substances other than collagen.

The invention thus includes an article, composition, or device having a body made of matrix material that includes insoluble collagen fibrils, and disposed within the body:
(a) a plurality of vertebrate cells (particularly mammalian cells such as cells derived from a human, chimpanzee, mouse, rat, hamster, guinea pig, rabbit, cow, horse, pig, goat, sheep, dog, or cat);
(b) one or more agents (see below) associated with (e.g., bound to, coated onto, or encapsulated by) a solid substrate; and
(c) a plurality of microcarriers, each of which preferably consists primarily of (i.e., greater than 50% of its dry weight is) one or more substances selected from a list including collagen (preferably type I collagen), polystyrene, dextran, polyacrylamide, cellulose, calcium alginate, latex, polysulfone, glass (e.g., glass coated with a gel such as collagen, to improve adherence of cells), and gelatin (e.g., porous gelatin). Generally at least 70%, and preferably at least 80% (most preferably between approximately 90% and approximately 100%, e.g., at least 95%) of each microcarrier's dry weight is one or more of the listed substances. Commercial examples of microcarriers that are described as consisting essentially of purified collagen include ICN Cellagen™ Beads and Hyclone Gelatin Cultispheres. The microcarriers are preferably of a porous consistency, but can be smooth, and typically have an approximately spherical shape with a diameter of approximately 0.1 to 2 mm (e.g., between approximately 0.3 and 1 mm). Of course, the shape and size of microcarriers from any particular lot or preparation will vary within manufacturing tolerances.

The article can be configured to be implanted into an animal, e.g., a mammal such as a human patient, or can be designed for producing cellular products *in vitro*; e.g., in an extracorporeal bioreactor apparatus having a means for shunting blood from an animal to the article and then back into a blood vessel of the animal, or in a bioreactor or other vessel from which medium containing the desired cellular product can be recovered for purification and the preparation of a pharmaceutical agent.

The cells can be derived from one or more cells removed from the patient, and preferably are genetically engineered (e.g., transfected) cells containing exogenous DNA encoding one or more polypeptides (e.g., medically useful polypeptides) such as an enzyme, hormone, cytokine, colony stimulating factor, angiogenesis factor, vaccine antigen, antibody, clotting factor, regulatory protein, transcription factor, receptor, or structural protein. Examples of such polypeptides include human growth hormone (hGH), Factor VIII, Factor IX, erythropoietin (EPO), albumin, hemoglobin, alpha-1 antitrypsin, calcitonin, glucocerebrosidase, low density lipoprotein (LDL) receptor, IL-2 receptor, globins, immunoglobulins, catalytic antibodies, the interleukins, insulin, insulin-like growth factor I (IGF-1), insulinotropin, parathyroid hormone (PTH), leptin, an interferon (IFN) (e.g., IFN-α, IFN-β, or IFN-γ), nerve growth factors, basic fibroblast growth factor (bFGF), acidic FGF (aFGF), epidermal growth factor (EGF), endothelial cell growth factor, platelet derived growth factor (PDGF), transforming growth factors, endothelial cell stimulating angiogenesis factor (ESAF), angiogenin, tissue plasminogen activator (t-PA), granulocyte colony stimulating factor (G-CSF), granulocyte-macrophage colony stimulating factor (GM-CSF), follicle stimulating hormone (FSH), α-galactosidase,β-gluceramidase, α-iduronidase, iduronate 2-sulfatase, glucosamine-N-sulfatase, α-N-acetylglucosaminidase, acetylcoenzyme A:α-glucosaminide-N-acetyltransferase, N-acetylglucosamine-6-sulfatase, β-galactosidase, N-acetylgalactosamine-6-sulfatase, β-glucuronidase, vascular endothelial growth factor (VEGF), VEGF-A, VEGF-B, VEGF-C, and VEGF-D. Alternatively, the exogenous DNA can contain a regulatory sequence, and optionally other elements, that will activate expression of an endogenous gene (for example, using homologous recombination as described in WO94/12650-PCT/US93/11704).

Generally any type of cell that is capable of attaching to collagen and/or the microcarriers, and that exhibits a desirable property such as expression of a medically useful cellular product or performance of an essential structural or metabolic function, can be utilized in the matrices of the invention. Examples include adipocytes, astrocytes, cardiac muscle cells, chondrocytes, endothelial cells, epithelial cells, fibroblasts, gangliocytes, glandular cells, glial cells, hematopoietic cells, hepatocytes, keratinocytes, myoblasts, neural cells, osteoblasts, pancreatic beta cells, renal cells, smooth muscle cells and striated muscle cells, as well as precursors of any of the above. If desired, more than one type of cell can be included in a given matrix. The cells can be present as clonal or heterogenous populations.

The collagen in the matrix material is preferably type I, but can be any other type of collagen. The matrix material can optionally include two or more types of collagen (e.g., selected from types I, II, III, IV, V, VI, VII, VIII, IX, X, and XI), as well as any additional components that impart desirable characteristics to the resulting matrix: e.g., agarose, alginate, fibronectin, laminin, hyaluronic acid, heparan sulfate, dermatan sulfate, chondroitin sulfate, sulfated proteoglycans, fibrin, elastin, tenascin, heparin or polysaccharides such as cellulose, starch, dextran, or chitosan. Furthermore, either instead of collagen or in addition to collagen, the following substances can be included in the matrices, either to endow upon them the properties of collagen or to enhance such properties: minced adipose tissue, minced omental tissue, methyl cellulose, alginate, gelatin, or fibrin. Any of the above-mentioned collagenous and non-collagenous components can be derived from human sources or from another animal or plant source. If from an animal source and potentially immunogenic, it is preferably the same animal as the subject into which it is to be implanted. One could also include collagen or non-collagen fibers disposed within the device. Collagen fibers can be in the form of cross-linked collagen threads dispersed within the body of the matrix material. Non-collagen fibers can, for example, be made of a material that includes nylon, dacron, polytetrafluoroethylene, polyglycolic acid, polylactic/polyglycolic acid polymer mixtures, polystyrene, polyvinylchloride co-polymer, cat gut, cotton, linen, polyester, or silk.

Large numbers of cells can be contained within the hybrid matrices. For example, hybrid matrices can be prepared which contain at least approximately two (and preferably approximately three) times as many cells as matrices prepared with soluble collagen alone, assuming the number of cells inoculated and the initial production volume are equivalent. The total amount of the polypeptide (e.g., a medically useful polypeptide) expressed by the cells embedded in a given hybrid matrix in a given time period is typically significantly higher (e.g., at least 50% higher, preferably at least 100% higher, and more preferably at least 200% higher) than achieved with a standard collagen matrix prepared from an equivalent volume of starting material.

The hybrid matrices of the invention also contain one or more (e.g., at least 2, 3, 4, 5, 6, 8, or 10) agents intended to improve the functioning of the matrix, e.g., by increasing proliferation and/or maintenance of the cells. These agents can include, for example, factors that promote vascularization, cytokines, or growth factors. While the agent used in a particular hybrid matrix and the polypeptide (e.g., a medically useful polypeptide) produced by the cells in the matrix can be the same substance, the two entities will generally be different. The agent is associated with (e.g., bound to, coated onto, or encapsulated within) a solid substrate that is added to the same mixture. The solid substrate can be the microcarriers themselves or can be a separate entity or entities (e.g., multiple particles of the solid substrate, or a single piece, embedded in the matrix). The solid substrate can have heparin or heparan sulfate proteoglycan bound to it, as a means for promoting binding of the agent. An example of such a solid substrate is one that consists primarily of agarose (e.g., Sepharose®, Affi-Gel™, Heparin Gel, or Heparin-agarose), with or without heparin or heparan sulfate proteoglycan bound to it; such a solid substrate can also contain calcium alginate. Other substances from which the solid substrates can be manufactured include collagen, gelatin, ethylene-vinyl acetate, polylactide/glycolic acid co-polymer, fibrin, sucrose octasulfate, dextran, polyethylene glycol, an alginate, polyacrylamide, cellulose, latex, polyhydroxyethylmethacrylate, nylon, dacron, polytetrafluoro-ethylene, polyglycolic acid, polylactic acid, polystyrene, polyvinylchloride co-polymer, cat gut, cotton, linen, polyester, and silk. The solid substrate can be in a variety of physical forms, e.g., beads, irregular particles, sheets, or threads. When the agent is encapsulated in the solid substrate, the agent is released gradually over time. These solid substrates can function as microcarriers as well as agent reservoirs. Thus, where a particular matrix of the invention includes solid substrates that can function as microcarriers, it is not necessary that it contain any of the above-listed microcarriers.

Examples of agents that can be used in the matrices include basic fibroblast growth factor (bFGF), VEGF, VEGF-A, VEGF-B, VEGF-C, VEGF-D, acidic fibroblast growth factor (aFGF), endothelial cell growth factor, platelet-derived growth factor (PDGF), endothelial cell stimulating angiogenesis factor (ESAF), leukotriene C₄, a prostaglandin, insulin-like growth factor 1 (IGF-1), granulocyte colony stimulating factor (G-CSF), angiogenin, transforming growth factor-_{α} (TGF-_{α}), transforming growth factor-β (TGF-β), ascorbic acid, epidermal growth factor (EGF), oncostatin M, or angiopoietin 1, 2, 3 or 4.

The bioactive concentration of each agent will vary greatly. A starting range is provided by the manufacturer and is usually based on a standard bioactivity assay using, for example, degree of cell proliferation as the end point. Typically, the agent may be bound at any of a broad range of concentrations (lowest being what is reported as bioactive by the vendor, highest being as much as 1000x of the reported concentration) to a substrate such as heparin-Sepharose® beads; the beads are incorporated into an HCM; and the release of the agent over time *in vitro* is monitored using an appropriate detection system (e.g., an immunoassay). For these release assays, the matrices are placed in growth medium containing 10% serum. Once it is determined that the matrices release detectable amounts of the agent, a bioactivity assay is performed. Matrices containing a range of agent concentrations can, for example, be placed on porous inserts (3 to 8µm pores) above cells that are known to proliferate in response to the agent (e.g, endothelial cells for VEGF and bFGF, fibroblasts for bFGF and PDGF, as indicated by manufacturer), and cell growth curves determined. The results of the *in vitro* bioactivity assay are evaluated and doses that are not deemed bioactive as well as doses that are determined to be "toxic" (i.e., lead to cell numbers lower than control) are noted. In order to determine the optimal concentration of agent per matrix, matrices containing the agent at a range of concentrations based on the *in vitro* bioactivity results can be implanted in immunocompromised mice. The optimal agent concentration per matrix is typically the concentration that allows for the maximum amount of therapeutic protein to be released for the maximum amount of time *in vivo*.

Instead of (or in addition to) the described agent being in the body of the matrix material bound to, or encapsulated within, a solid substrate, the hybrid matrices can contain, in addition to the first population of cultured vertebrate cells genetically engineered to express a polypeptide (e.g., a medically useful polypeptide), a second population of cultured vertebrate cells expressing and secreting one or more (e.g., at least 2, 3, 4, 6, 8, or 10) of the agents. The cultured vertebrate cells of the second population can be genetically engineered (as described below) to express the agent, or can be a cell which produces the agent without the benefit of genetic engineering. In the latter case, if the cell does not constitutively produce the polypeptide, or produces it in very low amounts, the cell can be induced to produce the agent, or produce it higher amounts, by gene activation. The matrices of the invention can contain additional populations of cultured vertebrate cells which express and secrete additional examples of the above described agents. The cultured vertebrate cells of the agent-producing populations can be, for example, adipocytes, astrocytes, cardiac muscle cells, chondrocytes, endothelial cells, epithelial cells, fibroblasts, gangliocytes, glandular cells, glial cells, hematopoietic cells, hepatocytes, keratinocytes, myoblasts, neural cells, osteoblasts, pancreatic beta cells, renal cells, smooth muscle cells, striated muscle cells, or precursors of any of the above. The cells are preferably human cells, but can be cells of any vertebrate (e.g., a mammal such as a non-human primate, pig, cow, horse, goat, sheep, dog, cat, mouse, rat, rabbit, guinea pig or hamster). When cells producing the agent are included in the matrix, one can optionally include the solid substrate as well, to bind a portion of the agent as it is secreted from the cells. This provides a means for controlling the rate of release of the agent from the hybrid matrix.

In one preferred embodiment, the hybrid matrices of the invention contain keratinocytes, e.g., (a) as the cells which produce the polypeptide (e.g., a medically useful polypeptide), (b) as the cells which produce the agent, (c) both (a) and (b), or (d) as a population which produces neither the polypeptide nor the agent. Hybrid matrices to which the keratinocytes are added are preferably those containing fibroblasts as cells producing the above-described polypeptide (e.g., a medically useful polypeptide) and/or agent. The keratinocytes and the fibroblasts can be obtained from the same individual, and one or more keratinocyte differentiation factors (e.g., calcium ions at a concentration of 1.5-2mM, TGF-β, or keratinocyte differentiation factor-1 (KDF-1)) can be added to the hybrid matrix.

In a similar manner, the hybrid matrices of the invention can contain endothelial cells, preferably in addition to fibroblasts producing a polypeptide (e.g., a medically useful polypeptide), and even in addition to both fibroblasts and keratinocytes. The endothelial cells and fibroblasts can be obtained from the same individual. One or more endothelial differentiation factors (e.g. VEGF or bFGF at 10 ng - 10µg) can be added to the hybrid matrix to induce the formation of endothelial tubes within the matrix. The differentiation factors can be added directly to the matrix during formation, or added to the matrix growth medium, or both.

The hybrid matrix of the invention is generally prepared by a process that includes the following steps:
forming a mixture that includes (a) a plurality of vertebrate cells; (b) a plurality of microcarriers, each of which preferably consists primarily of one or more substances selected from the list consisting of collagen, polystyrene, dextran, polyacrylamide, cellulose, calcium alginate, latex, polysulfone, and glass; (c) a solution comprising soluble collagen; and (d) one or more of the above-described agents associated with (e.g., bound to, coated onto, or encapsulated within) a solid substrate; and
causing the soluble collagen in the mixture to form a gel of insoluble collagen fibrils in which the cells and the microcarriers are embedded; and
exposing the gel to culture conditions which cause the gel to become smaller by the exclusion of liquid, thereby forming the body of the article. Gelation is typically triggered by raising the pH of the relatively acidic collagen solution to above 5, e.g., by addition of concentrated, buffered culture medium, whereupon the collagen forms insoluble fibrils. When this step is carried out in a mold, the gel will take the shape of the interior of the mold. Generally the contraction of the gel is effected by the cells in the mixture, which attach to the fibrils and cause the gel to contract to a smaller version of the molded shape (e.g., a disk, as in the case where the mold is a petri dish which is cylindrical in shape). The matrix can be utilized immediately after manufacture, can be cultured to increase the number of cells present in the matrix or to improve their functioning, or can be cryopreserved indefinitely at a temperature below 0°C. They can also be stored temporarily in a higher temperature refrigerator at, for example, about 4°C.

For making those hybrid matrices containing one or more of the above-described agents, the relevant agents (bound to or encapsulated within any of the above-described solid substrates) are added, together with the other components listed above, to the mixture. The agent and solid substrate can be added to the mixture together or separately, in any order. Additionally or alternatively, the mixture can contain a second (and, optionally, a third, fourth, fifth, sixth, or more) population of cultured vertebrate cells secreting one or more of the described agents. The mixture can also contain one or more of the above-described solid substrates that include one or more substances (e.g., heparin or heparan sulfate) which binds to an agent. For making hybrid matrices containing keratinocytes, the keratinocytes can be added to the mixture prior to the contraction step, or they can be added to the body of the composition after the gel has contracted.

In making any of the hybrid matrices of the invention, the gel can be formed in a flat-bottomed mold filled with the mixture to a depth of about 0.18 cm (e.g., about 0.1 cm to about 0.3 cm, preferably about 0.15 cm to about 0.21 cm). For example, the gel can be formed in a flat-bottomed cylindrical mold having an internal radius (r) using a mixture having a volume (V), such that r²/V is about 1.8 (e.g., 1.5 to 2.0). The invention includes hybrid matrices resulting from use of the specified depth of mixture, and/or the specified ratio of r²/V, and thus having a characteristic thickness. The gel can be formed, for example, in a cylindrical mold having a radius of about 2.65 cm, using a volume of 4 ml, or in a mold the radius of which is other than 2.65 cm (i.e., larger or smaller), with a proportional change in volume of mixture used.

A polypeptide (e.g., a medically useful polypeptide), such as one listed above, can be delivered to a patient by a treatment method that involves providing a hybrid matrix containing cells which secrete the polypeptide of interest, and implanting the article in the patient in a selected site, such as a subcutaneous, intraperitoneal, intraomental, sub-renal capsular, inguinal, intramuscular or intrathecal site. Where the polypeptide is one that promotes wound healing (e.g., PDGF or IGF-I), the matrix can be implanted at the site of a preexisting wound. As discussed above, the cells can be derived from one or more cells removed from the patient, and are preferably transfected *in vitro* with exogenous DNA encoding the polypeptide. Alternatively, they can be cells which naturally secrete the polypeptide or perform the desired metabolic function (e.g., hepatocytes or pancreatic beta cells). The cells can be induced by gene activation to secrete the polypeptide, secrete higher amounts of the polypeptide, or perform the desired metabolic function. Appropriate hybrid matrices for delivering a polypeptide to a patient can be any of those described above.

In another embodiment, the polypeptide (e.g., a medically useful polypeptide) can be administered to the patient by shunting a portion of the patient's blood through the apparatus described above, so that the polypeptide secreted by the cells in the hybrid matrix mixes with the blood. Generally, any such apparatus known to those in that field can be adapted to accommodate the matrix of the invention. For example, blood shunted into a device which contains a perm-selective membrane surrounding a matrix of the present invention will result in the delivery of a therapeutic product of the matrix to the blood. A device similar to an artificial pancreas (Sullivan et al., Science 252:718-721, 1991) can be used for this purpose. Again, any of the hybrid matrices described herein can be used for such devices.

Yet another use for any of the hybrid matrices of the invention is as a means for producing a desired polypeptide (e.g., any of the polypeptides listed herein) *in vitro*. This method includes the steps of placing the hybrid matrix under conditions whereby the cells in the matrix express and secrete the polypeptide; contacting the matrix with a liquid such that the cells secrete the polypeptide into the liquid; and obtaining the polypeptide from the liquid, e.g., by standard purification techniques appropriate for the given polypeptide. In one embodiment, the matrix is anchored to a surface and is bathed by the liquid; alternatively, the matrix floats freely in the liquid. Cells embedded in the hybrid matrix function at a high level in a small space. Furthermore, the first step in purification of the expressed polypeptide (removal of the cells from the medium) is considerably more efficient with the matrices than with most standard methods of cell culture.

The invention also includes a fluid mixture that contains:
(a) a population of cultured vertebrate cells (e.g., any of the mammalian cells listed above) that generally express a polypeptide;
(b) a plurality of microcarriers such as those listed above; and
(c) at least one agent selected from the group consisting of a factor which promotes vascularization, a cytokine, a growth factor, and ascorbic acid, the at least one agent being associated with (e.g., bound to, coated onto, or encapsulated within) a solid substrate suspended in the collagen solution.

The cells can be any of those described above for use in matrices and can be similarly genetically engineered to express the same polypeptides.

The collagen in the mixture can be any of the types listed herein or a combination of any of these types. There is sufficient collagen in the mixture to allow the mixture to gel at a pH above 5. The fluid mixtures can also contain any of the additional components listed above. Instead of collagen or in addition to collagen, the following substances can be included in the mixtures, either to endow upon matrices generated from the mixtures the properties of collagen or to enhance such properties: minced adipose tissue, minced omental tissue, methyl cellulose, alginate, gelatin, and fibrin.

The microcarriers permit high concentrations of cells to be contained within the mixtures of the invention. For example, when soluble collagen is employed so that the implanted mixture forms an *in situ* hybrid matrix, such a hybrid matrix will have the advantages of hybrid matrices formed *in vitro* (see above).

The mixtures of the invention can also contain one or more (e.g., at least 2, 3, 4, 5, 6, 8, or 10) of the above-described agents intended to improve the functioning of the matrix generated from the mixtures, e.g., by increasing proliferation and/or maintenance of the cells. Moreover, the agents can be added to the mixture in any of the forms described for matrices, e.g., free in solution or coated onto solid substrates.

Methods for determining bioactive concentrations of each agent for use in mixtures of the invention are essentially the same as those described above for matrices. In addition, however, the mixtures can be introduced into the mice so that hybrid matrices are formed *in situ* before making the described *in vivo* measurements.

Instead of (or in addition to) the described agent *per se* being added to the mixture, the mixtures can contain, in addition to the first population of cultured vertebrate cells expressing a polypeptide (e.g., a medically useful polypeptide), any of the second population of cultured vertebrate cells described above in regard to the matrices of the invention.

In one preferred embodiment, the mixtures of the invention contain keratinocytes which can be obtained, play the same roles, and function in the same way as those in matrices. In addition, the mixtures can contain the same keratinocyte differentiation factors listed above. Moreover, as described for matrices, the mixtures of the invention can contain endothelial cells, and, optionally, the above-described endothelial differentiation factors.

The mixture of the invention is generally prepared by combining, in an aqueous solution, (a) a population of any of the above-described cells; and (b) a plurality of any of the above-described microcarriers. Soluble collagen, or one or more of the alternatives listed above, is preferably also added to the mixture.

For making those mixtures containing one or more of the above-described agents, the relevant agents (bound to, or encapsulated within, any of the above-described solid substrates) are added, together with the other components listed above, to the mixture. The agent and solid substrate can be added to the mixture together or separately, in any order. Additionally or alternatively, a second (and, optionally, a third, fourth, fifth, sixth, or more) population of cultured vertebrate cells secreting one or more of the described agents can also be added to the mixture. Furthermore, one or more of the above-described solid substrates that include one or more substances (e.g., heparin or heparan sulfate) which binds to an agent can be added to the mixture. Keratinocytes can also be added to the mixture.

A polypeptide (e.g., a medically useful polypeptide) such as one listed above can be delivered to a patient by a treatment method that involves providing any of the above mixtures of the invention that contains cells (e.g., cells secreting a polypeptide of interest), and introducing the mixture into the patient in a selected site, such as a subcutaneous, intraperitoneal, intraomental, sub-renal capsular, inguinal, intramuscular or intrathecal site. Where the polypeptide produced by the cells is one that promotes wound healing (e.g., PDGF or IGF-1), the mixture can be introduced at the site of a preexisting wound. Introduction of the mixture into-a patient can be by any means appropriate for the liquid suspension: e.g., a pipette, a catheter or a hypodermic syringe optionally capped with a syringe needle or a catheter. The invention includes a method in which introduction of the mixture into an animal occurs at substantially the same time as forming the mixture from its components (e.g., the cultured vertebrate cells, microcarriers, and collagen). As discussed above, the cells can be derived from one or more cells removed from the patient, and are preferably transfected *in vitro* with exogenous DNA encoding the polypeptide. Alternatively, they can be cells which naturally secrete the polypeptide or perform the desired metabolic function (e.g., hepatocytes or pancreatic beta cells). The cells can be induced to secrete the polypeptide, secrete higher amounts of the polypeptide, or perform the desired metabolic function by gene activation. Appropriate liquid mixtures for delivering a polypeptide to a patient can be (a) any of those described above; or (b) any of those described above but lacking microcaniers.

The invention also includes a vessel containing any of the above-described liquid mixtures of the invention. The vessel can be, for example, a glass or plastic hypodermic syringe, bottle, test tube, flask, or vial. It can also be a plastic bag, e.g., a blood bag. The vessel can be used, for example, for transporting a mixture of the invention from a site of manufacture or retail sale to a site of use, e.g., a hospital or a doctor's or veterinarian's office. Gelation prior to introduction of the mixture into a patient can be inhibited by, for example, maintaining the contents of the vessel at a temperature below about 8°C. Where another type of vessel is used, e.g., a vial or a bottle, the mixture can be transferred to an apparatus that is suitable for introducing the mixture into the patient (e.g., a hypodermic syringe or a blood bag) before substantial gelation has occurred. Such a vessel of the invention can be included, together with shipping material (e.g., a shipping container), in a kit.

The invention also features a kit that includes a shipping container containing:
(a) a first vessel containing
   (i) a population of cultured vertebrate cells that express a polypeptide (e.g., cells genetically engineered to express the polypeptide), and
   (ii) a plurality of microcanriers;
(b) a second vessel containing a collagen solution; and
(c) a third vessel containing a liquid medium.

There will be sufficient collagen in the collagen solution to allow a mixture formed by mixing the contents of the vessels of (a), (b), and (c) to gel. The collagen solution can be at an acidic pH and the medium can be at an alkaline pH. The mixture to be introduced into an animal is prepared from the kit by (a) combining the contents of the second and third vessels to form a collagen/medium solution; and (b) combining the collagen/medium solution with the contents of the first vessel to form the mixture. Step (b) and introduction of the liquid mixture into the subject should occur shortly after the collagen/medium solution is formed, i.e., prior to gelation of the collagen to the point the mixture is no longer a liquid. The acidic pH (in the range of about pH 2.0 to about pH 4.0) of the collagen solution in the second vessel prevents gelation of the collagen during storage. Thus, gelation of the mixture to form a solid matrix does not occur until after neutralization of the acidic collagen solution with the alkaline medium to a pH of about 7.2 to about 7.8, preferably about 7.4 to about 7.8. Alternatively, the collagen solution in the second vessel can be at a pH of about 7.2 to about 7.8, preferably about 7.4 to about 7.8, with gelation inhibited by maintaining the collagen solution at a temperature below 10°C (e.g., at a temperature of about 3°C to 8°C) until immediately prior to mixing and introduction into the animal. In either case, after formation of the mixture, it is immediately introduced into an animal, i.e., before polymerization has proceeded beyond the point the mixture can be readily transferred into the implantation site. The kit will preferably include packaging material and instructions for use in accordance with the methods of the invention.

Alternatively, the kit can include a shipping container containing two vessels with
(a) the first vessel containing
   (i) a population of cultured vertebrate cells that express a polypeptide (e.g., cells genetically engineered to express the polypeptide); and
   (ii) a plurality of microcarriers; and
(b) the second vessel containing a collagen solution.

There will be sufficient collagen in the collagen solution to allow a mixture formed by mixing the contents of the vessels of (a) and (b) to gel. The collagen solution can be at an acidic pH. In this case, the pH of the collagen solution can be adjusted to about 7.2 to about 7.8, preferably about 7.4 to about 7.8, immediately prior to formation of the mixture, using an alkaline solution, e.g., a solution of NaOH at a concentration of about 0.01N to about 1.0N. The kit can optionally include (1) a third vessel containing such an alkaline solution, and/or (2) a pH indicator such as pH paper, a dipstick, or a dye-containing solution. Alternatively, the collagen solution in the second vessel can include a pH-indicator dye.

In another embodiment, the collagen solution in the second vessel is at a pH of about 7.2 to about 7.8, preferably about 7.4 to about 7.8, with gelation inhibited by keeping the contents of the second vessel below about 8°C until immediately before formation of the mixture. Again, formation of the mixture would generally be followed immediately by introduction of the mixture into an animal, unless the mixture is maintained below 8°C to prevent gelation.

In each case, the kit will preferably include appropriate packaging material and labels and/or an insert detailing instructions for use. The packaging material can include, for example, a container suitable for shipping the mixture components to a retailer or a site of use, e.g., a hospital or a physician's office. Suitable shipping containers include, for example, plastic (or other synthetic polymer), styrofoam, cardboard, wood, or metal boxes. Paper or plastic (or other synthetic polymer) bags, envelopes, or blister packs can also be used as shipping containers. The shipping containers can also include impact protective materials such as plastic (e.g., polystyrene) fibers or chips, bubble wrap, or shredded paper, or molded plastic with slots adapted to hold the vessels, and can be thermally insulated.

The vessels used in such kits can be glass or plastic hypodermic syringes, bottles, test tubes, flasks, or vials. They can also be plastic bags, similar to blood bags. They can be supplied in a thermally insulated container in order to maintain one or more of the components at a constant temperature, e.g., a temperature below about 8°C.

In these kits, the collagen can be any of the types listed herein. Furthermore, the collagen can be substituted or supplemented with any of the alternative substances described herein. The cultured vertebrate cells can be, for example, any of those listed, and they can have been derived and/or genetically engineered by any of the procedures described herein. The polypeptides expressed by the cells and the microcarriers can be, for example, any of those listed above. The noncollagen fibers indicated above can be present in any one or more of the vessels.

The kits can further contain in any of the vessels one or more (e.g., two, three, four, five, six, seven, eight, nine, or ten) agents, e.g., a factor that promotes vascularization, a cytokine, a growth factor, or ascorbic acid. The agents can be, for example, any of those listed herein. Examples of solid substrates with which the agents are optionally associated are described above. Also optionally present in a vessel of the kit is a second, third, fourth, fifth, sixth, seventh, eighth, ninth, or tenth population of cultured vertebrate cells secreting one or more (e.g., two, three, four, five, six, seven, eight, nine, or ten) agents, e.g., a factor that promotes vascularization, a cytokine, or a growth factor, as described above.

Where the kits include fibroblasts as one of the populations of cultured vertebrate cells, a vessel of the kit can further contain endothelial cells and/or keratinocytes and, optionally, a keratinocyte differentiation factor (e.g., calcium ions at a concentration of 1.5-2 mM), as described above. The fibroblasts and the endothelial cells and/or keratinocytes can have been obtained, for example, from the same individual.

The invention also features an apparatus for introduction of a mixture into a patient. The apparatus includes at least two (e.g., two, three, four, five, or six) vessels, each of which has an outlet in fluid communication with a delivery means. The apparatus can contain (a) an aqueous collagen solution in which the collagen can be any of the collagens listed herein, (b) a population of cultured vertebrate cells that express any of the polypeptides disclosed herein (e.g., cells genetically engineered to express any such polypeptide), and (c) a plurality of any of the microcarriers listed above. One or more of (a), (b), or (c) can be contained within each vessel. There is sufficient collagen in the collagen solution to allow a mixture formed by mixing components (a), (b), and (c) to gel. One or more of the vessels can have associated with it a piston having two ends, a first end adapted to be inserted into the interior of the vessel, and a second end adapted to extend from the interior of the vessel. The delivery means can be a syringe needle or catheter or other tube having (a) two or more inlets adapted to be in fluid communication with the outlets of the vessels, and (b) an outlet. Alternatively, the delivery means can be an adapter piece having a connector in fluid connection with the outlet of each of the vessels, a mixing chamber, and an outlet in fluid communication with a syringe needle, catheter, or other tube; the connector can contain at least two inlets (e.g., two, three, four, five, or six) each in fluid communication with the outlet of only one of the vessels. The apparatus can further include one or more connections physically joining at least two vessels. In addition, the second end of each piston can be connected to a pressure plate. The apparatus can also include any of the other cell populations, alternatives to collagen, non-collagen fibers, or agents recited herein.

As used herein, a "solid substrate" is an object, or a plurality of objects (configured, for example, as particles or threads), which acts as a reservoir or depot for a substance (e.g., a factor that promotes vascularization) that is contained within or is bound to the solid substrate. The substance is gradually released from the solid substrate into its environment. Where the solid substrate is in the form of beads, the beads have, generally, an approximately spherical shape and have a diameter of approximately 0.005 - 2.0 mm. Where the solid substrate is in the form of threads, the threads are generally about 0.01 - 1.0 mm in diameter. The threads can be woven, folded into a meshwork or cut into small pieces (of approximately 5 -10 mm) prior to gel formation. Where the threads are folded, their length should be, for example, about 1x to 3x (e.g., about 2x) the diameter of a mold used to produce the relevant hybrid matrix. As used herein, a "solid substrate" is not a cell.

Unless otherwise defined, all technical and scientific terms used herein have the same meaning as commonly understood by one of ordinary skill in the art to which this invention pertains. In case of conflict, the present document, including definitions, will control. Preferred methods and materials are described below, although other methods and materials similar or equivalent to those described herein can be used in the practice or testing of the present invention. The materials, methods, and examples described herein are illustrative only and not intended to be limiting.

Other features and advantages of the invention are apparent from the claims, and from the detailed description provided below.

### Brief Description of the Drawings

Fig. 1 is a map of hFVIII expression plasmid pXF8.198.
Fig. 2 is a line graph showing levels of human factor VIII (hFVIII) in plasma from mice implanted with Heparin-Sepharose Hybrid Collagen Matrices (HSHCM) containing cells producing hFVIII and heparin-Sepharose® beads either coated with basic fibroblast growth factor (bFGF) or uncoated.
Fig. 3 is a line graph showing levels of hFVIII in plasma from mice implanted with HSHCM containing cells producing hFVIII, one of two types of microcarriers, and heparin-Sepharose® beads either coated with bFGF or uncoated.
Fig. 4 is a line graph showing levels of hFVIII in plasma from mice implanted with HSHCM containing cells producing hFVIII and heparin-Sepharose® beads coated with various concentrations of bFGF.
Fig. 5 is a line graph showing levels of hFVIII in plasma from mice implanted with HSHCM containing cells producing hFVIII and either heparin-Sepharose® beads coated with bFGF, heparin-Sepharose® beads coated with vascular endothelial growth factor (VEGF), or a mixture of heparin-Sepharose beads® coated with bFGF and heparin-Sepharose® beads coated with VEGF.
Fig. 6 is a diagram depicting the pXVEGF.1 plasmid.
Fig. 7 is a line graph showing levels of hFVIII in plasma from mice implanted with HSHCM containing a first population of cells producing hFVIII, either alone or together with second population of cells producing VEGF.
Fig. 8 is a line graph showing the levels of hFVIII in plasma of mice injected subcutaneously with either 1mL or 2mL I-HSHCMM containing cells producing hFVIII and heparin-Sepharose® beads coated with bFGF.
Fig. 9 is a map of hFVIII expression plasmid pXF8.186
Fig. 10 is a line graph showing the levels of hFVIII in plasma of mice injected intraomentally with either cells producing hFVIII, cells producing hFVIII and heparin-Sepharose® beads coated with bFGF (50 ng), or cells producing hFVIII and heparin-Sepharose® beads coated with VEGF (100 ng).
Fig. 11 is a line graph showing the levels of hFVIII in plasma of mice injected intraomentally with either cells producing hFVIII, cells producing hFVIII and heparin-Sepharose® beads coated with bFGF (500 ng), or cells producing hFVIII and heparin-Sepharose® beads coated with VEGF (100 ng).
Fig. 12 is a line graph showing the levels of hFVIII in plasma of mice injected intraomentally with cells producing hFVIII and either microcarriers, microcarriers and heparin-Sepharose® beads, microcarriers and heparin-Sepharose® beads coated with bFGF (500 ng), or microcarriers and heparin-Sepharose® beads coated with VEGF (100 ng).
Fig. 13A is a diagram of an apparatus that can be used to simultaneously form a mixture of the invention and deliver it to a subject.
Fig. 13B is a cross-sectional view of the adapter piece 4 component of the apparatus depicted in FIG. 13A.

### Detailed Description

The examples set forth below illustrate several embodiments of the invention. These examples are for illustrative purposes only, and are not meant to be limiting.

### EXAMPLE I

### General Description of Heparin-Sepharose Hybrid Collagen Matrices (HSHCM)

HSHCM are produced by mixing together concentrated Dulbecco's Modified Eagle's Medium (DMEM), collagen (e.g., rat tail type I or a suitable alternative, for example, human placental type I or III collagen), microcarriers (for example, collagen macroporous microcarriers as described above or porous gelatin microcarriers), heparin-Sepharose® beads either uncoated or coated with an angiogenic factor, a cytokine, or a growth factor (for example, basic fibroblast growth factor (bFGF), vascular endothelial growth factor (VEGF), or platelet derived growth factor (PDGF)), and cells expressing the therapeutic protein. In an alternative embodiment, multiple cell strains are mixed into the matrix, with one strain expressing the therapeutic protein of interest and another strain expressing the angiogenic factor, cytokine, or growth factor. It is also possible to utilize a single strain that expresses both the therapeutic protein and the angiogenic or growth factor. In this embodiment, the heparin-Sepharose® bead component can be uncoated or coated with the same or a different growth factor from the one expressed by the incorporated cell strain, or it can be omitted altogether.

### Microcarrier Preparation

un The preparation of collagen microcarriers for matrix production is as follows. Collagen microcarriers (Cellex Biosciences cat. #YB00-0015UW) were transferred from each original bottle provided by the manufacturer (∼10ml per bottle) into 50 ml conical tubes (1 tube per bottle). The microcarriers were allowed to settle in the tube, and the storage buffer solution was aspirated off. Microcarrier wash medium (DMEM with 1% calf serum and 1% penicillin/streptomycin) was added to the 50 ml mark on the graduated tube, the microcarriers allowed to settle, and the medium aspirated off. This series of washing steps was repeated for a total of 4 washes. The microcarriers were transferred to a 250 ml Erlenmeyer flask using a 25 ml plastic pipette, limiting the volume of microcarriers to 100 ml per 250 ml flask. Microcarrier wash medium was added to the 250 ml mark, and the flask was capped and placed in a tissue culture incubator at 37°C for 2-3 hours. The flask was removed from the incubator, the microcarriers allowed to settle, and the wash medium aspirated off. This series of incubation and washing steps was repeated for a total of 3 washes.

The preparation of gelatin microcarriers was performed as follows. One gram of dry Cultisphere-GL™ gelatin microcarriers (Hyclone Catalog # DG-1001-00) was placed in a 100ml glass bottle containing 50ml of phosphate-buffered saline without calcium or magnesium (PBS). The gelatin microcarriers were allowed to hydrate for at least an hour at room temp. They were sterilized by autoclaving at 121°C for 15 min. The gelatin microcarriers were conditioned for use in matrices by removing the PBS and washing 3x in DMEM + 1% calf serum + 1% Penicillin-Streptomycin (50ml per wash). The gelatin microcarriers were swirled gently and allowed to settle between washes. Conditioned gelatin microcarriers can be stored at 4°C prior to use.

### Heparin-Sepharose® Bead Preparation

Heparin coupled to Sepharose® agarose beads provides a solid support for the attachment of heparin-binding growth factors such as bFGF, VEGF, and PDGF. Sepharose® is a highly cross-linked agarose resin manufactured by Pharmacia Biotech and is a registered trademark of the manufacturer. Heparin linked to a different type of support matrix (for example, a polymer) can be used instead of a heparin-agarose bead. Heparin-Sepharose® 6 Fast Flow beads (Pharmacia Biotech Cat #17-0998-03) were washed 3x in dH₂O at 5x the bead volume per wash. The beads were swirled gently after addition of dH₂O and centrifuged at 500_{*xg*} between each wash step. They were sterilized by autoclaving at 121°C for 20 min. The beads were equilibrated at pH 7.2_ 7.4 by extensive washing in PBS until the pH of the 1:1 bead to PBS slurry was within the range of 7.2-7.4. The beads were stored at 4°C in 1:1 slurry with PBS.

Angiogenic and growth factors were bound to the heparin-Sepharose® beads as follows. Equilibrated beads were placed in an appropriate size sterile tube (for example, a 1.5ml Eppendorf or a 15ml conical tube) and centrifuged at 500_{*xg*} for 4 min. They were washed 2x in PBS, with a centrifugation step between each wash. The angiogenic or growth factor (e.g., at 50µg/ml in the case of bFGF) was added to the beads in a volume of PBS equal to the packed bead volume to give a 1:1 bead to PBS slurry, and the binding was performed under rotation (for example, on a Nutator™ platform) at room temp. for 1h. The PBS containing any unbound growth factor was removed from the beads by aspiration, and the beads were washed 1x in PBS at a volume equal to the packed bead volume. The PBS was removed, and the beads were placed on ice until matrix formation.

### HSHCM Preparation

The different components used to prepare HSHCM are listed in Tables 1 and 2. These tables describe production of HSHCM using collagen microcarriers (Table 1) or gelatin microcarriers (Table 2). Each table gives the volume of each component per ml of production medium, as well as the volume necessary for production of 10 x 4ml HSHCM as an example. (Volumes and concentrations given for matrices hereinbelow refer to the pre-contraction volume and concentration, respectively.) The matrix "pre-mix" consists of 10X DMEM, 7.5% NaHCO₃, 1M HEPES, Penicillin-Streptomycin, L-glutamine, and dH₂O. The pre-mix was generally prepared an hour or two prior to mixing with collagen, cells and the angiogenic factor-coated beads, and kept at 4°C. Cells to be embedded in the HSHCM were harvested by trypsinization and the cell number was determined. The required number of cells (cell number per matrix multiplied by the total number of matrices produced) was centrifuged at 1500 rpm (500_{*xg*}) for 7 min at room temperature. The cell pellet was resuspended in a volume of growth medium equal to 10% of the total volume of HSHCM production medium as indicated in Tables 1 and 2. The density of cells per ml matrix can vary as appropriate (e.g., 0.1_10 x 10⁶ cells per ml matrix). Since the cell suspension was added at a volume that is 10% of the total production medium volume, the number of cells per ml of this suspension was 10x greater than the desired density of cells per ml matrix (e.g., 1_100x10⁶ cells per ml). The appropriate volume of microcarriers is placed in a conical tube, and the cell suspension was added to the microcarriers. Using a 10ml plastic pipette, the cells and microcarriers were mixed together and set aside at room temp. for approximately 10 to 15 minutes prior to incorporation into the production medium. The pre-mix was placed on ice and a solution of collagen (e.g., rat tail collagen (RTC)) was added to the chilled pre-mix and mixed thoroughly using a 10ml plastic pipette. The pH of the production medium was then adjusted to approximately 7.8 by addition of 1N NaOH at the volume indicated in Table 1 or 2. The cell/microcarrier mixture was added to the neutralized production medium and mixed using a 10ml plastic pipette. A volume of growth medium equal to the packed volume of heparin-Sepharose® beads was added to the beads, and the beads were transferred to the production medium and mixed using a 10ml plastic pipette. A volume of this final HSHCM production medium was added to the appropriate size Petri dish. For example, 4ml of HSHCM production medium is generally added to a 60mm Petri dish, 10ml to a 100mm Petri dish, and 30ml to a 150mm Petri dish. Varying the ratio of production medium volume to dish surface area can modify the thickness of the HSHCM. The dishes containing the HSHCM production medium were transferred to an incubator with a temperature of 37°C, humidity of 95 to 98%, and a CO₂ level of 5%. One hour later, the dishes were removed from the incubator and examined. If the HSHCM production medium had gelled, the HSHCM were fed by addition of 5ml of growth medium. Otherwise, the dishes were returned to the incubator for an additional hour or until polymerization was complete, at which time the HSHCM were fed by addition of 5 ml of growth medium. The growth medium was supplemented with 10-100 ng/ml of the same growth factor or angiogenic factor used to coat the heparin-Sepharose® beads of the HSHCM.

**TABLE I**

| HSHCM Production Media Formulation: Collagen Microcarrier Configuration. | | | |
|---|---|---|---|
| Component | Volume (per ml of production medium) | Total Vol. for 10 x 4ml matrices | Final Conc. |
| 10X DMEM | 0.1ml | 4.0ml | 1X |
| 7.5% NaHCO₃ | 0.012ml | 0.48ml | 0.9g/L |
| 1M HEPES | 0.0025ml | 0.1ml | 2.5mM |
| Pen/Strep (100X stock) | 0.01ml | 0.4ml | 1% |
| L-glutamme (200mM stock) | 0.01ml | 0.4ml | 2mM |
| dH₂O | 0.16ml | 6.4ml | 16% |
| RTC (4mg/ml, 0.02N acetic acid) | 0.25ml | 10ml | 1mg/ml |
| 1N NaUH (to pH 7.4-7.8) | 0.005ml | 0.2ml | 0.5% |
| Collagen microcarriers* | 0.25ml | 10ml | 25% |
| Cell suspension* | 0.1ml | 4.0ml | 10% |
| Heparin-Sepharose® beads (as a 1:1 slurry in DMEM + 10% calf serum)* | 0.1ml | 4.0ml | 5.0% dry weight |
| Total Volume | 1.0ml | 40.0ml | |

| | | | |
|---|---|---|---|
| *Added to the production medium after pH is adjusted | | | |

**TABLE 2**

| HSHCM Production Media Formulation: Gelatin Microcarrier Configuration. | | | |
|---|---|---|---|
| Component | Volume (per ml of production medium) | Total Vol. for 10 x 4ml matrices | Final Cone. |
| 10X DMEM | 0.1ml | 4.0ml | 1X |
| 7.5% NaHCO₃ | 0.012ml | 0.48ml | 0.9g/L |
| 1M HEPES | 0.0025ml | 0.1ml | 2.5mM |
| Pen/Strep (100X stock) | 0.01 ml | 0.4ml | 1% |
| L-glutamme (200mM stock) | 0.01ml | 0.4ml | 2mM |
| dH₂O | 0.15ml | 6.0ml | 15% |
| RTC (4mg/ml, 0.02N acetic acid) | 0.438ml | 17.5ml | 1.75mg/ml |
| 1N NaOH (to pH 7.4-7.8) | 0.015ml | 0.6ml | 1.5% |
| Gelatin microcarriers* | 0.0625ml | 2.5ml | 6.25% |
| Cell suspension* | 0.1ml | 4.0ml | 10% |
| Heparin-Sepharose® beads (as a 1:1 slurry in DMEM + 10% calf serum)* | 0.1ml | 4.0ml | 5.0% dry weight |
| Total Volume | 1.0ml | 40.0ml | |

| | | | |
|---|---|---|---|
| * Added to the production medium after pH is adjusted | | | |

### In Vitro Maintenance and Evaluation of HSHCM

HSHCM were maintained in culture by feeding the matrices on day 1, and then 2 to 3 times weekly using the following protocol:
1. Carefully aspirate the culture medium.
2. Add the required volume of appropriate growth medium, taking into consideration the size of the dish used for each matrix. The medium can be supplemented with ascorbic acid 2-phosphate and/or TGF-β (e.g. 10-50 µg/ml ascorbic acid 2-phosphate and/or 1-10 ng/ml TGF-β). The volume of growth medium used to feed the matrices is usually the maximum volume that can be added to each dish size, for example, 10ml per 60mm dish, 20-30ml per 100mm dish, and 100_150ml per 150mm dish.

Matrix diameter size and cell number per matrix were determined as follows.

The diameter of a HSHCM can be determined using the following procedure:
1. Place the petri dish containing the matrix to be measured on top of a metric ruler resting on a dark background.
2. Record diameters (in centimeters) as desired: e.g., daily for the first 2 weeks, every other day after the first 2 weeks, and on days of cell quantitation for the duration of the experiment. C. Cells can be recovered and quantified from a HSHCM as follows:

1. Enzymatic Digestion of Hybrid Collagen Matrices
   a. Prepare a solution of collagenase IA (1.0 mg/ml for HSHCM, 2.0 mg/ml for HSHCM supplemented with ascorbic acid 2-phosphate and/or TGF-β) in PBS.
   b. Dispense the collagenase solution into 15 ml conical centrifuge tubes at a volume of 1 ml for matrices seeded with 1 - 5x 10⁶ cells and 5 ml for matrices seeded with greater than 5x10⁶ cells per matrix. Prepare as many collagenase tubes as there are matrices to be counted.
   c. Remove each matrix from its dish using flat forceps, and carefully blot the excess fluid from the matrix using an absorbent paper towel (if cells will be discarded after counting) or sterile absorbent pad.
   d. Place each matrix in an individual collagenase-containing tube, cap tightly, and secure onto an orbital shaker set at 40 rpm in a tissue culture incubator.
   e. Incubate the matrices for approximately 1 hour or until digestion is complete. To accelerate digestion, break up the matrices by pipetting at 5 min intervals.
2. Cell Counting
   a. Measure the total volume of each digested cell suspension using the gradations on the side of the centrifuge tube.
   b. For measuring cell viability, remove 0.1 ml of cell suspension and add to 0.1 ml of 0.08% trypan blue in a 1.5 ml microcentrifuge tube. Mix by tapping the tube lightly.
   c. Count the viable and dead cells using a hemacytometer. If necessary, further dilute the cell suspension in PBS prior to adding the trypan blue. Calculate the total number of cells, taking into consideration the total volume measured in step 2a.

### EXAMPLE II

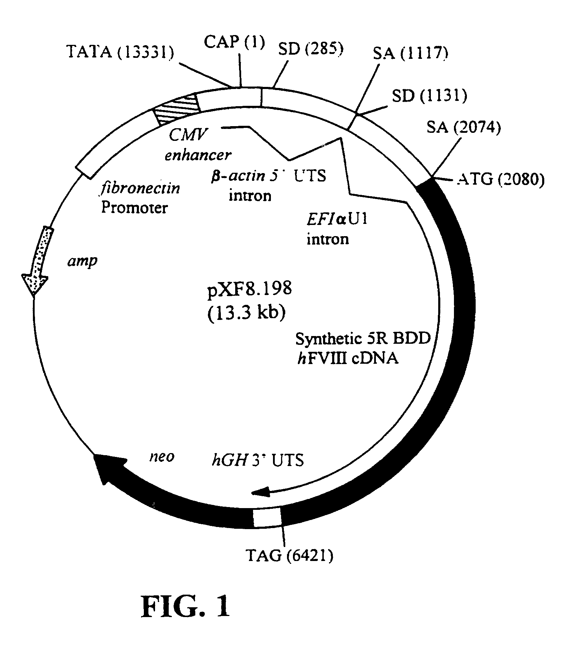

This example describes *in vivo* implantation of HSHCM prepared as described in Example I. HSHCM containing either uncoated heparin-Sepharose® beads or heparin-Sepharose® beads coated with bFGF (50µg/ml packed beads; 10µg total bFGF/matrix) were prepared as described in Table 1. Each 4ml matrix was formed with 5 x 10⁶ cells of HF743 B1-35, a human foreskin fibroblast clone containing plasmid pXF8. 198 (Fig. 1) and expressing hFVIII at levels between 20,000 _30,000 mU/24h/10⁶ cells. Further detailed procedures for preparing and transfecting cells suitable for use in the matrices and mixtures of this invention are provided in WO93/09222 (PCT/US92/09627), which is incorporated herein by reference in its entirety. The HSHCM were maintained in culture for 2 days prior to implantation. For subcutaneous implantation of matrices, *Rag*-*2* mice (129S6/SvEvTac-[KO]*Rag2*, Taconic Farms) were anesthetized and prepped as follows. The mice were given an intraperitoneal injection of Avertin**™** (solution of 2% w/v 2,2,2-tribromoethanol and 2% v/v 2-methyl, 2-butanol) at a dose of 0.0173 ml/g body weight. Anesthetized mice were placed in lateral recumbency, and the implant site was shaved and disinfected with alcohol and Betadine™ (0.75% Iodine solution). A 2 cm incision was made in the cutaneous layer on the left side of the animal, inferior and caudal to the ribs, and the fascia between the cutaneous and external oblique muscle layer was cleared and enlarged using sterile blunt scissors. HSHCM were prepared for implantation by washing 2x in 10 ml of PBS. Each matrix was folded into quarters using sterile spoon-shaped spatulas, and inserted into the cleared cutaneous space using a sterile spatula. The incision was closed using wound clips.

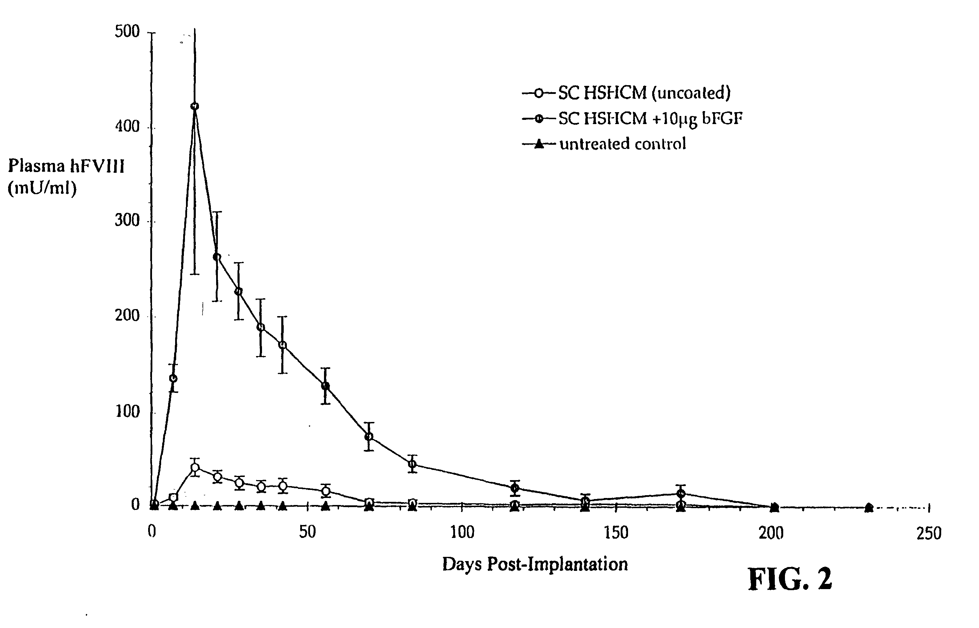

Blood was collected by retroorbital bleed after placing the mouse in a large beaker containing methoxyflurane (Pittman-Moore) until light anesthesia was achieved. Plasma hFVIII was determined using a hFVIII ELISA based on two mouse monoclonal antibodies [Homsey et al. (1992) Transfus. Med. 2(3):223-229]. The estimated number of cells per matrix was determined by digesting and counting recovered cells from HSHCM set aside for this purpose. HSHCM containing uncoated heparin-Sepharose beads had an average of 2.8 x 10⁶ cells per matrix and HSHCM containing bFGF-coated heparin-Sepharose beads had an average of 3.1 x 10⁶ cells per matrix (n = 3 matrices per condition). The *in vitro* production of hFVIII from each matrix type on the day of implantation was 71,165 mU/24h and 85,657 mU/24h from HSHCM containing uncoated and bFGF-coated beads, respectively (n = 3 matrices per condition). As shown in Fig. 2, HSHCM containing bFGF-coated heparin-Sepharose beads led to a significantly higher plasma level of hFVIII detected in the host mice as compared to HSHCM without the growth factor (n = 10 mice per condition). There was no detectable hFVIII on day 0, and the peak plasma level occurred on day 14.

The following examples describe additional studies involving subcutaneous implantation of HSHCM (prepared as described in Example I) into *Rag-2* mice, and utilize the HF743-B1-35 hFVIII secreting cell clone introduced above.

### EXAMPLE III

HSHCM containing either uncoated heparin-Sepharose beads or heparin-Sepharose beads coated with bFGF (50µg/ml packed beads, 10µg total bFGF/matrix), and either collagen microcarriers or gelatin microcarriers, were prepared as described in Tables 1 and 2, respectively. The average number of cells per matrix and hFVIII production per matrix on the day of implantation (n = 3 matrices per condition) are listed in Table 3.

**TABLE 3**

| Summary of cell number and hFVIII production per HSHCM on the day of implantation. | | |
|---|---|---|
| Condition | Cell# (x 10⁶) | hFVIII (mU/24h/HSHCM) |
| Collagen microcamers & uncoated heparin-Sepharose® beads | 4.2 | 84,989 |
| Gelatin microcarriers & uncoated heparin-Sepharose® beads | 3.9 | 94,407 |
| Collagen microcarriers & bFGF-coated heparin-Sepharose® beads | 4.3 | 138,612 |
| Gelatin microcarriers & bFGF-coated heparin-Sepharose® beads | 3.5 | 107,683 |

Subcutaneous (SC) cell controls were included in this experiment and were prepared as follows. Cells were harvested by trypsinization in the same manner as for preparation of HCM. The trypsinized cells were counted, centrifuged at 500*xg*, resuspended in Hank's Buffered Saline Solution (without calcium and magnesium), centrifuged, and resuspended in a volume of PBS to give 100 x 10⁶ cells/ml based on the original cell count after harvest. The cells in the cell/PBS slurry were counted, and the density of cells in the suspension was adjusted to 50 x 10⁶ cells/ml PBS. For each injection, a total of 0.15ml of cell slurry was aspirated into a 1cc Glasspak™ syringe and the syringe was capped with a 22G/1 inch needle. The cell slurry was injected SC into the left side of the mouse, inferior and caudal to the ribs. Taking into consideration the void volume of the needle, a total of 0.1ml, or 5 x 10⁶ cells, was injected into each control animal (n = 5 mice per condition).

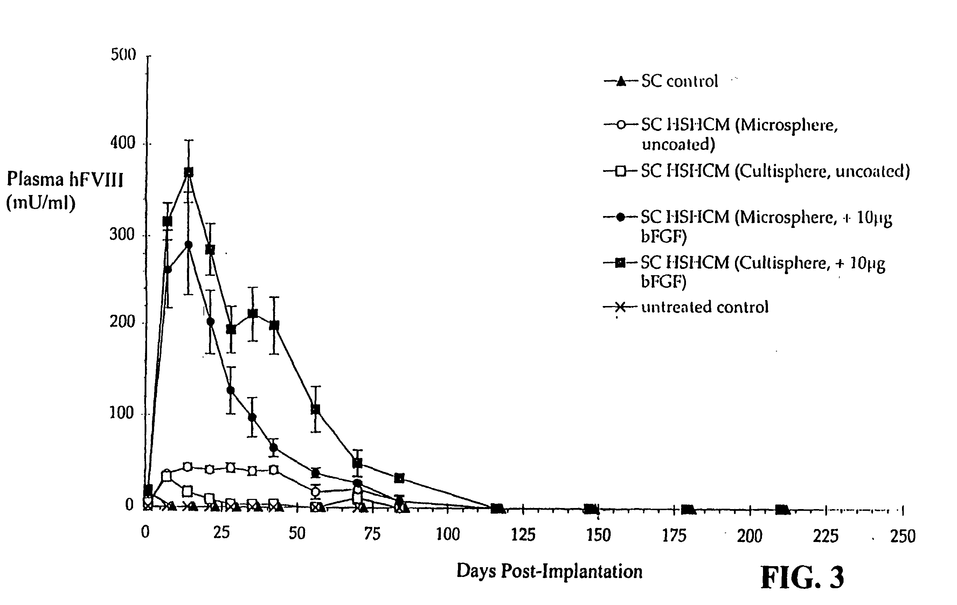

Fig. 3 shows that the HSHCM containing the gelatin microcarriers and bFGF-coated heparin-Sepharose® beads led to a significantly higher level of hFVIII when implanted into mice, compared to the other conditions (n = 10 mice per condition). The expression pattern was similar to that observed in Example II, with no detectable hFVIII until day 7, and highest expression at day 14. There was no detectable hFVIII in the plasma of the mice receiving SC cell injections at any time point after day 1 post-implantation.

### EXAMPLE IV

HSHCM containing heparin-Sepharose® beads either uncoated or coated with bFGF at 12.5, 25, or 50µg/ml packed beads were prepared using the collagen microcarrier formulation (Table 1). The total amount of bFGF per matrix after incorporation of the coated beads was estimated to be approximately 2, 4, and 10µg, respectively. The average number of cells per matrix and hFVIII production per matrix on the day of implantation (n = 3 matrices per condition) are summarized in Table 4.

**TABLE 4**

| Summary of cell number and hFVItI production per HSHCM on the day of implantation. | | |
|---|---|---|
| Condition | Cell # (x 10⁶) | hFVIII (mU/24h/HSHCM) |
| Uncoated heparin-Sepharose**®** beads | 3.8 | 62,956 |
| bFGF at 2µg/HSHCM | 7.0 | 92,836 |
| bFGF at 4µg/HSHCM | 10.9 | 80,976 |
| bFGF at 10µg/HSHCM | 8.2 | 97,964 |

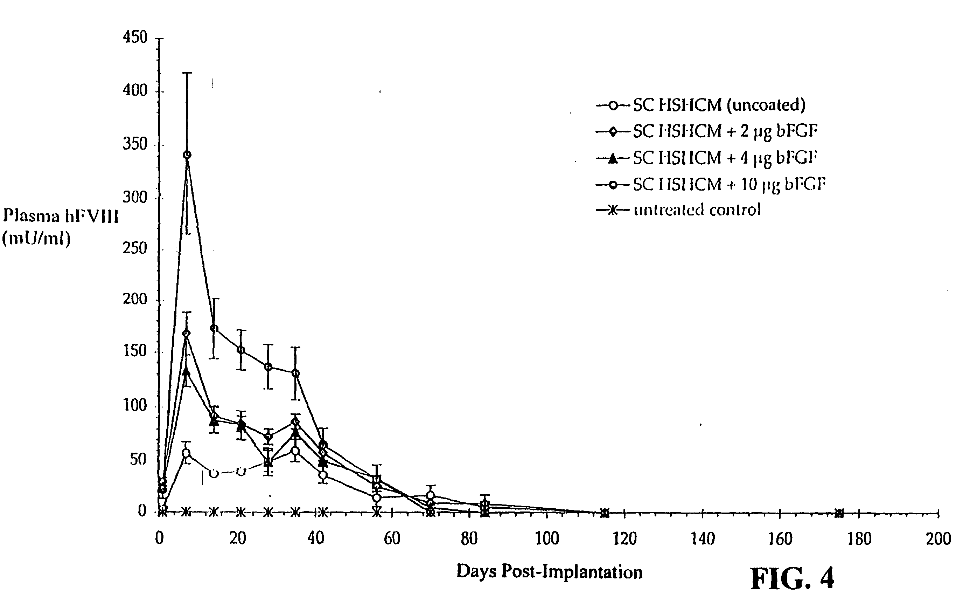

Fig. 4 demonstrates that plasma levels of hFVIII are significantly higher following implantation of matrices containing 10µg of bFGF as compared to those containing 4µg and 2µg of bFGF (n = 5 mice per condition). The pattern of expression was similar to that observed in Examples II and III.

### EXAMPLE V

HSHCM containing heparin-Sepharose beads either uncoated, coated with bFGF, coated with VEGF, or coated with both bFGF and VEGF were prepared according to the gelatin microcarrier formulation (Table 2). For the set of HSHCM containing beads coated with both growth factors, each matrix received 0.1ml of beads coated with bFGF and 0.1ml of beads coated with VEGF in order to keep the bead volume consistent with previous experiments. The coating concentration of each growth factor for this condition was 100µg/ml packed beads, resulting in a total of approximately 10µg of each growth factor per HSHCM. In the HSHCM+bFGF+VEGF there were approximately 10µg each of bEGF and VEGF. The average number of cells per matrix and hFVIII production per matrix on the day of implantation (n = 3 matrices per condition are summarized in Table 5.

**TABLE 5**

| Summary of cell number and hFVIII production per HSHCM on the day of implantation. | | |
|---|---|---|
| Condition | Cell # (x 10⁶) | Hfviii (mU/24h/HSHCM) |
| Uncoated heparin-Sepharose® beads | 3.7 | 101,048 |
| HSHCM + bFGF | 3.5 | 128,654 |
| HSHCM + VEGF | 3.4 | 76,152 |
| HSHCM + bFGF + VEGF | 4.9 | 127,450 |

Intraomental (IO) implantation controls were included in order to compare the subcutaneous delivery of hFVIII from HSHCM with another implantation method that has proven successful for the delivery of hFVIII in the mouse model. IO controls were prepared as follows. Cells were harvested by trypsinization in the same manner as for preparation of HCM. The trypsinized cells were counted, centrifuged at 500*xg*, resuspended in Hank's Buffered Saline Solution (without calcium and magnesium), recentrifuged, and resuspended in a volume of PBS to give 15 x 10⁶ cells/ml based on the original cell count after harvest. The cells in the cell/PBS slurry were counted, and a volume of cell suspension equal to 5 x 10⁶ cells was distributed into sterile 1.5ml microcentrifuge tubes and centrifuged at 500*xg* for 4 min in a microcentrifuge. The tubes containing the cell pellet and PBS were placed on ice, and each cell pellet was implanted into the omental recess of the *Rag-2* mouse in the following manner. Prior to implantation of cells, mice were anesthetized with 1.25% Avertin™ and placed in lateral recumbency. The left flank between the ribs and stifle was wiped with an alcohol pad and prepped with Betadine™. A small (0.5 cm to 1.0 cm) incision was made posterior to the ribs and ventral to the spine. The spleen was exposed and gently exteriorized. Cells were injected along the axis of the spleen upon the cranial and medial aspect and within the thin membrane adjacent to the hilar surface of the spleen. The spleen was replaced in the abdominal cavity, and the incision closed.

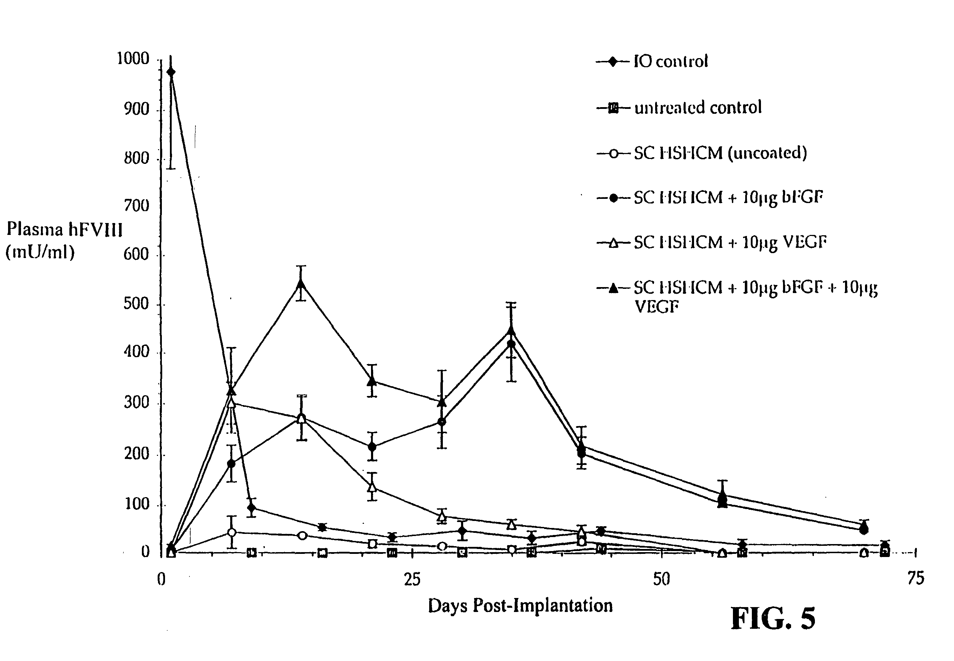

Fig. 5 demonstrates that the plasma hFVIII levels are significantly higher from HSHCM containing a combination of bFGF and VEGF as compared to the other conditions at days 14 and 21 (n = 5 mice per condition). The injection of cells IO led to a significantly higher plasma hFVIII level on day 1, but by the next time point (day 7), the level observed in IO controls fell below that obtained with the HSHCM implants containing growth factors (n = 5 mice per condition).

### EXAMPLE VI

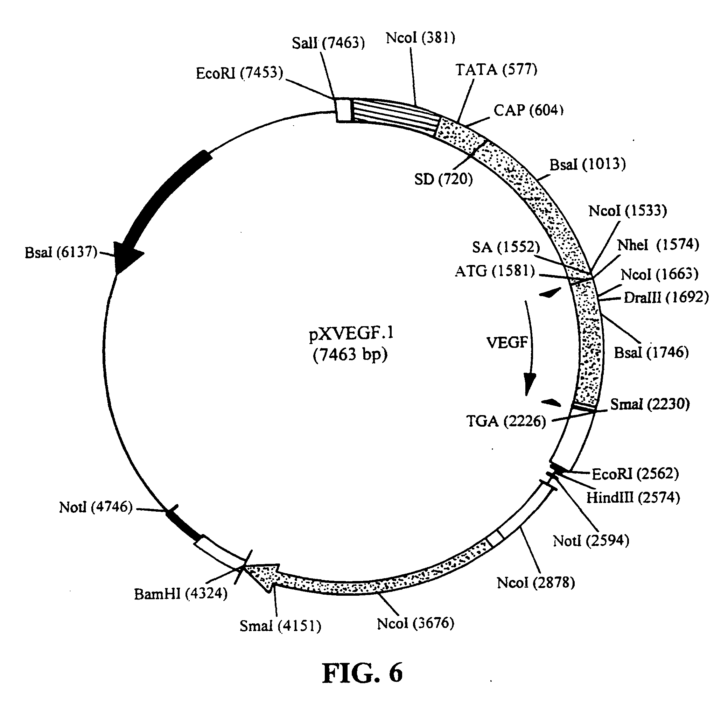

HSHCM containing a combination of 2 fibroblast cell clones, HF743 B1-35 expressing hFVIII and HF811-M15 containing plasmid pXVEGF.1 (Fig. 6) and expressing VEGF [Kock et al., Science 246:1309-1312, 1989], were prepared for implantation. The HF811-M15 clone was producing an average of 85 ng VEGF/24h/10⁶ cells at the time of implantation. The matrices were prepared according to the collagen microcarrier formulation (Table 1). The heparin-Sepharose® bead component was uncoated. Two sets of HSHCM were prepared with one or the other clone at 5 x 10⁶ cells per matrix. An additional two sets were prepared with a constant number of HF743 B1-35 cells (5 x 10⁶) and an additional 1 x 10⁶ or 2.5 x 10⁶ of HF811-M15 cells. Table 6 summarizes the different conditions and *in vitro* production levels of hFVIII and VEGF for each condition (n = 5 matrices per condition).

**TABLE 6**

| Summary of hFVIII and VEGF production per HSHCM on day of implantation. | | |
|---|---|---|
| Condition (cells/matnx) | hFVIII (mU/24h) | VEGF (ng/24h) |
| HF743 B1-35 (5 x 10⁶) | 65,442 | n/a |
| HF 743 B1-35 (5 x 10⁶) + HF811-M15 (1 x 10⁶) | 79,828 | 47 |
| HF743 B1-35 (5 x 10⁶) + HF811-M15 (2.5 x 106) | 63,876 | 86 |
| HF811-M15 (5 x 10⁶) | n/a | 82 |

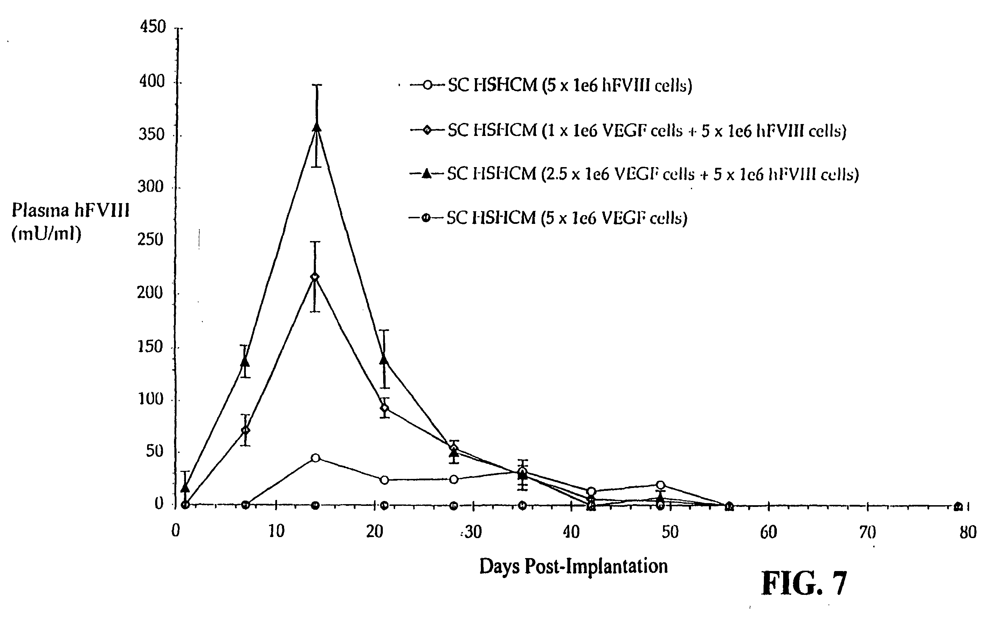

Plasma hFVIII levels over time for each HSHCM condition are illustrated in Fig. 7. The set of HSHCM containing 2.5 x 10⁶ HF811-M15 cells led to the highest hFVIII plasma levels, which peaked on day 14 and declined until day 42, at which time no hFVIII was detectable. The HSHCM containing 1 x 10⁶ HF811-M15 cells led to plasma levels of hFVIII slightly lower than the set containing 2.5 x 10⁶ HF811-M15, but significantly higher than the HSHCM without the VEGF-producing cells, indicating that VEGF produced a dose-dependent effect on the delivery of hFVIII by the implant.

The amount of VEGF in the plasma samples was determined by ELISA (R&D Systems VEGF Immunoassay Kit). The results are summarized in Table 7. There was a decline in detectable VEGF from day 14 to day 28 in plasma from mice implanted with HSHCM made with either 2.5 x 10⁶ or 5 x 10⁶ HF811-M15 cells. VEGF levels were not detectable in the plasma of animals implanted with HSHCM made without HF811-M15 cells or with 1 x 10⁶ HF811-M15 cells. Plasma samples for day 1 and 7 were not available for assay, but the data suggest that the VEGF level peaked at some point between time of implantation and day 14.

**TABLE 7**

| Summary of VEGF expression in the plasma of *Rag-2* mice implanted with HSHCM containing different numbers of HF811-M15 cells. | | | | |
|---|---|---|---|---|
| Condition (cells/matrix) | Plasma VEGF (pg/ml) | | | |
| | Day 14 | Day 21 | Day 28 | Day 35 |
| HF 743 B1-35 (5 x 10⁶) | 0 | 0 | 0 | 0 |
| HF 743 B1-35 (5 x 10⁶) + HF811-M15 (1 x 10⁶) | 0 0 0 0 | | | |
| HF743 B1-35 (5 x 10⁶) + HF811-M15 (2.5 x 10⁶) | 6.5 | 1.4 | 0 | 0 |
| HF811-M15 (5 x 10⁶) | 89 | 77 | 0 | 0 |

### EXAMPLE VII

The volume of the mixture used to make the matrices of the invention is based on a liquid column height (i.e., depth) of 0.18 cm. A 4 ml volume of mixture in a 60mm dish (radius 2.65 cm) was found to give optimal cell viability and protein expression for that size dish. Based on the formula V =πr²h, where V = volume of the mixture, r = radius of the dish, and h = height of the liquid column (i.e., depth of the mixture in the dish), the optimal volume of 4 ml = π(2.65 cm)²h, so optimal depth = 0.18 cm and optimal r²/V ratio = 1.8. The following table (Table 8) indicates optimal volumes for different dish sizes, including the 60mm size from which the 0.18 cm height was derived.

**TABLE 8**

| Optimal volumes and r²/V ratios for different size dishes. | | | | | |
|---|---|---|---|---|---|
| Dish Size (mm) | Diameter (cm) | Radius r(cm) | r² | Volume* (ml) | Ratio of r² to volume |
| 35 | 3.6 | 1.8 | 3.24 | 1.8 | 1.8 |
| *60* | *5.3* | *2.65* | *7.02* | *4* | *1.8* |
| 100 | 8 | 4 | 16 | 9 | 1.8 |
| 150 | 13.8 | 6.9 | 47.6 | 27 | 1.8 |

| | | | | | |
|---|---|---|---|---|---|
| * based on equation: volume = _{π}r^{z} x 0.18 cm | | | | | |

While a ratio of about 1.8 was found to be optimal for cell viability and protein expression, ratios of about 1.0 - 3.0, preferably 1.5 to about 2.0, were adequate. These correspond to depths of about 0.21 cm and about 0.16 cm, respectively.

### EXAMPLE VIII

The HSHCM of the invention would be prepared for implantation in humans as follows:

The desired cells, typically stably transfected autologous cells derived from the patient, are harvested from tissue culture dishes and processed for the production of HSHCM by any of the methods described above. The dosage for a given patient (i.e., the physiologically effective quantity of therapeutic product produced by the matrix) can be varied by using a larger or smaller matrix, implanting a different number of matrices into the patient, and/or using cells which express a different level of the product per cell when constructing the matrix. The quantity of the therapeutic product produced in the patient can also be varied by exposing the cells in the matrix to a pharmacologic or physiologic signal which alters expression of the therapeutic gene. For example, if the therapeutic gene is under the control of a glucocorticoid-responsive promoter, then *in vivo* exposure of the cells to a drug such as dexamethasone (by administering the drug to the patient in a manner that ensures the drug reaches the implant) will alter expression of the therapeutic gene.

Typically, a plurality of small matrices (approximately 1-2 cm) in diameter, produced in 60 mm petri dishes and containing on the order of 10 x 10⁶ cells per matrix, would be implanted. Thus, approximately 100 million cells could be implanted using 10 small matrices. The use of matrices with significantly higher cell densities (as produced by incorporating, for example, ascorbic acid 2-phosphate) would result in a smaller number of matrices needed for a given patient. Alternatively, a larger petri dish (> 150 mm diameter) can be used as a mold to produce larger matrices which could be either implanted directly or cut into smaller pieces which are implanted.

Prior to implantation, the matrices can be stored or shipped in growth medium or any other solution which allows the cells to remain viable. Alternatively, the matrices can be cryopreserved by freezing in an appropriate freezing medium, which can be washed away prior to implantation.

Matrices can be implanted in a variety of sites, including, but not limited to, subcutaneous, intraperitoneal, intrasplenic, intraomental, inguinal, intrathecal, intraventricular, and intramuscular sites, as well as within lymph nodes or within adipose tissue. A surgical incision at the appropriate site is made, the matrix inserted, and the incision closed.

### EXAMPLE IX

There are a number of static and perfusion large scale *in vitro* culture systems that can be adapted for use in protein manufacture using cells maintained in hybrid matrices of the invention. The choices offer varying levels of facility in the necessary steps of feeding and medium harvest prior to purification of target proteins. Several are described below.
1. After formation and maturation of HSHCM in conventional petri plates (e.g. after 10-25 days incubation), a number of these HSHCM can be aseptically transferred to a sterile Microcarrier Spinner Flask (250-100 ml capacity). These HSHCM are produced and maintained under conditions which maximize the viable cell density within each matrix. Typically this requires between 5 and 20 ml medium for every one ml of matrix volume. The protein production medium is formulated to comprise a minimum of undefined components (e.g., serum), and can include added factors intended to maximize the output of protein production per cell. The spinner flasks are placed into a 37° ± 1 °C, 5% ± 1% CO₂ humidified incubator on a magnetic stir platform set for 30-70 rpm. After 1-3 days, the flask is transferred to a class 100 biological safety cabinet, and the production medium containing the expressed protein is aseptically drawn off without disturbing the settled matrices. An equivalent volume of fresh protein production medium is added to the flask, and the flask is returned to the stir platform within the incubator.
2. The matrices described in (1) above can be aseptically transferred into a 1-5L bioreactor (e.g, Brunswick) with a controllable stirring impeller shaft. Production medium level is set by the control system of the bioreactor. Medium harvesting and replenishment is controlled within a sterile closed loop system to minimize contamination.
3. For high volume HSHCM production, the matrices are formed from the constituent components and allowed to gel within a tissue culture roller bottle. The bottles are incubated in a static upright position until contraction of the matrix results in a free-floating structure (3-5 days). Growth medium is then replenished, and the bottles are gassed with 5% CO₂ and placed into a roller apparatus within a 37°C incubator. Growth medium is replenished every 2-3 days until HSHCM are mature (10-25 days total incubation), at which time the HSHCM are exposed to protein production medium. After 1-3 days, the bottles are transferred to a class 100 biological safety cabinet and the production medium containing the expressed protein is aseptically drawn off without disturbing the matrices. An equivalent volume of fresh protein production medium is added to the bottle, which is returned to the roller apparatus.
4. The constituents of the HSHCM can be aseptically introduced into sterile gas-permeable Teflon™ bags through a sealable port. The components are allowed to gel within the bag and take on the shape and conformation therein. Bags are incubated in 37° ± 1°C, 5% ± 1% CO₂ humidified incubators. As the HSHCM contract and reduce in volume, the growth medium volume is adjusted to compensate. Medium harvesting and replenishment is accomplished through sterile-connect tube systems built into the bags. The use of ported incubators and extended tubing would allow for the design of a cyclic harvest/feed pumping system that could eliminate the need for removing the bags from incubators during a production run.
5. The constituents of the HSHCM can be aseptically introduced into custom-designed thermoformed trays with a high volume capacity. The simplest conformation would be an open lidded rectangular tray with gas exchange capabilities designed for use in a CO₂ tissue culture incubator. Another design would include a closed loop system with ported access to the medium reservoir for controlled feeding and medium harvest, akin to a bioreactor chamber.

### EXAMPLE X

### General Description of Injectable Heparin-Sepharose® Hybrid Collagen Matrix Mixtures (I-HSHCMM)

I-HSHCMM are prepared by mixing together concentrated DMEM (without phenol red), rat tail type I collagen (or a suitable alternative, for example, human placental type I and/or III collagen); porous microcarriers (for example, collagen macroporous microcarriers or porous gelatin microcarriers); optional heparin-Sepharose® beads either uncoated or coated with an angiogenic or growth factor (for example, basic fibroblast growth factor (bFGF), vascular endothelial growth factor (VEGF), or platelet derived growth factor (PDGF)); and cells expressing the therapeutic protein. In an alternative embodiment, a combination of cell strains are mixed into the collagen solution, with one strain expressing the therapeutic protein of interest and the other strain expressing the angiogenic or growth factor. It is also possible to utilize a single strain that expresses both the therapeutic protein and the angiogenic or growth factor. In this embodiment, the heparin-Sepharose bead component can be uncoated or coated with the same or a different growth factor from the one expressed by the incorporated cell strain, or it can be omitted altogether. Injectable matrix mixtures of the invention include those without collagen or any of the collagen alternatives disclosed herein. Furthermore, mixtures without a source of angiogenic factors or growth factors are included in the invention. It is also understood that the mixtures can lack microcarriers, but generally such mixtures will include an agent such as an angiogenic factor, a cytokine, a growth factor, or ascorbic acid. While not a requirement, such agents can be associated with any of the solid substrates described herein. These solid substrates can function as microcarriers to which the cells adhere, and also as reservoirs of relevant agents.

### Microcarrier Preparation and Heparin-Sepharose® Bead Preparation

Microcarriers and Heparin-Sepharose® beads were prepared as described above in Example I. Serum proteins were removed by 3-5 washes with serum-free PBS prior to formation of the mixtures.

### I-HSHCMM Preparation

The different components used to prepare I-HSHCMM are listed in Table 9, which describes preparation of I-HSHCMM using gelatin beads as the microcarrier. The table gives the volume of each component per mL of production medium, as well as the volume necessary for production of 10 x 1mL and 10 x 2mL 1-HSHCMM as an example. The matrix "pre-mix" consists of 4X DMEM without phenol red, 7.5% NaHCO₃, 1M HEPES, Penicillin-Streptomycin, L-glutamine, and dH₂O. The pre-mix is usually prepared an hour or two in advance and kept at 4°C. Cells to be injected along with the matrix components are harvested from tissue culture vessels and centrifuged at 500*xg* for 10 min to obtain a cell pellet. The cell pellet is washed 1x in PBS, the PBS is removed, and the pellet is resuspended in 1X DMEM without phenol red in a volume equal to 10% of the total volume of the I-HSHCMM production medium as indicated in Table 9. The density of cells per mL matrix production medium can vary as appropriate (e.g. 0.1 - 10 x 10⁶ cells per mL injectable matrix). Since the cell suspension is added at a volume that is 10% of the total production medium volume, the number of cells per mL of this suspension should be 10x greater than the desired density of cells per mL matrix mixture (e.g., 1 -100 x 10⁶ cells per mL). The appropriate volume of microcarriers (e.g., Cultispheres) is placed in a conical centrifuge tube, and the cell suspension is added to the microcarriers. Using a 10mL glass pipette, the cells and microcarriers are mixed together and set aside at room temp. for approximately 10 to 15 minutes prior to incorporation into production medium. The pre-mix is placed on ice and a solution of collagen (e.g. rat tail collagen (RTC)) is added to the chilled pre-mix and mixed thoroughly using a 10mL glass pipette. The production medium is then adjusted to a pH of approximately 7.8 by addition of 1N NaOH at the volume indicated in Table 9. The cell/microcarrier mixture is pelleted by centrifugation at 500*xg* for 5 min, the supernatant is aspirated, and a volume of DMEM without phenol red is added to the tube to achieve a final volume equal to the volume of the cell suspension plus the volume of microcarriers as listed in Table 9. The cell/microcarrier suspension is added to the neutralized production medium and mixed using a 10mL glass pipette. A volume of DMEM without phenol red equal to the packed volume of heparin-Sepharose® beads is added to the beads, and the beads are transferred to the neutralized production medium and mixed using a 10mL glass pipette. The complete production medium is kept on ice until immediately prior to injection, at which time it is loaded into an appropriate size syringe (e.g., 10cc). After loading, the syringe is capped with a needle (e.g., 16 gauge) and injected into the desired site (e.g., subcutaneous).

**TABLE 9**

| Injectable HSHCMM Production Media Formulation | | | | |
|---|---|---|---|---|
| Component | Volume (per mL) | Total Vol. for 10 x 1mL solutions | Total Vol. for 10x 2mL solutions | Final Conc. |
| 4X DMEM | 0.25mL | 2.5mL | 5.0mL | 1X |
| 7.5% NaHCO₃ | 0.012mL | 0.12mL | 0.24mL | 0.9g/L |
| 1M HEFES | 0.0025mL | 0.025mL | 0.05mL | 2.5mM |
| dH₂O | 0.0315mL | 0.315mL | 0.63mL | 3.15% |
| RTC (4mg/mL, 0.02N acetic acid) | 0.438mL | 4.38mL | 8.76mL | 1.75mg/mL |
| 1N NaOH (to pH 7.4-7.8) | 0.004mL | 0.04mL | 0.08mL | 0.4% |
| Cultispheres* | 0.0625mL | 0.625mL | 1.25mL | 6.25% |
| Cell suspension* | 0.1mL | 1.0mL | 2.0mL | 10% |
| Heparin-Sepharose® beads (as a 1:1 slurry in DMEM without phenol red)* | 0.1mL | 1.0mL | 2.0mL | 5.0% dry weight |
| Total Volume | 1.0mL | 10.0mL | 20.0mL | |

| | | | | |
|---|---|---|---|---|
| * Added to the production medium after pH is adjusted | | | | |

### In Vitro Assessment of Gelled Mixture

A volume of the final I-HSHCMM production medium is added to the appropriate size Petri dish via injection from a syringe through a 16G needle as described above. As an example, 2mL of I-HSHCMM production medium are added to a 35mm Petri dish, 4mL to a 60mm Petri dish, 10mL to a 100mm Petri dish, and 30mL to a 150mm Petri dish. Varying the ratio of production medium volume to dish surface area can modify the thickness of the gelled HSHCM. The dishes containing the I-HSHCMM production medium are then transferred to an incubator with a temperature of 37°C, humidity of 95 to 98%, and CO₂ level of 5%. One hour later, the dishes are removed from the incubator and examined. If the l-HSHCMM production medium has gelled, the HSHCM are fed by addition of 5mL of DMEM without phenol red, otherwise, the dishes are returned to the incubator for an additional hour or until polymerization is complete.

HSHCM are maintained in culture for the desired amount of time. The volume of culture medium used to feed the matrices is usually the maximum volume that can be added to each dish size, for example, 5mL per 35mm dish, 10mL per 60mm dish, 20 - 30mL per 100mm dish, and 100 - 150mL per 150mm dish. The cell number, cell viability per matrix, and level of polypeptide production are determined as described above.

### EXAMPLE XI

This example describes the *in vivo* injection of the 1-HSHCMM liquid mixture prepared as described in Example X. An I-HSHCMM liquid mixture containing heparin-Sepharose® beads coated with bFGF at 100µg/mL packed beads for a 2mL injection volume and 200µg/mL packed beads for a 1mL injection volume (10µg total bFGF delivered per implant) was prepared as described in Table 9. For each injection volume, HF743 B 1-35, a human foreskin fibroblast clone containing plasmid pXF8.198 (Fig. 1) and expressing hFVIII at levels between 20,000 - 30,000 mU/24h/10⁶ cells, was incorporated, with microcarriers, to achieve a total of 5 x 10⁶ cells per injection. For subcutaneous introduction of the mixture, *Rag*-2 mice (129S6/SvEvTac-[KO]*Rag*-2, Taconic Farms) were anesthetized and prepped as described above. The implant site, located on the left side of the animal inferior and caudal to the ribs, was shaved and disinfected with alcohol and Betadine™. Immediately prior to injection, the complete I-HSHCMM liquid mixture was loaded into a 10cc syringe at a volume slightly greater than the desired injection volume to compensate for the void volume of the needle. The loaded syringe was capped with a 16G needle and the I-HSHCMM was immediately injected into the subcutaneous site. The injected solution spread out within the space between the cutaneous tissue and external oblique muscle. Slight pressure was applied to the point of injection and the needle was slowly pulled away from the skin. The injected solution began to solidify within a few seconds after injection. The mice were placed on a heat pad immediately after injection to prevent possibile hypothermia.

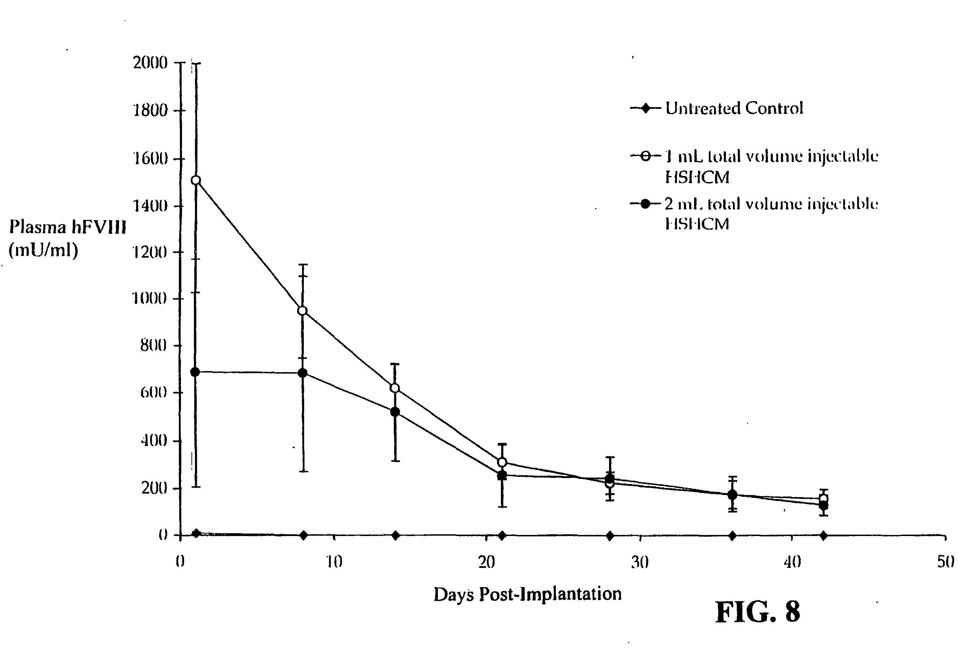

Blood was collected and plasma hFVIII was detetmined using a hFVIII ELISA based on two mouse monoclonal antibodies as described in Example II. The estimated number of cells per gelled matrix was determined by digesting and counting recovered cells from HSHCM formed in Petri dishes (2mL in 35mm dish) and set aside for this purpose. The HSHCM gels were kept in culture for 2 days prior to assessment of cell number and viability, and were maintained in serum-free DMEM as described in Example X. Gelled HSHCM (n = 3 matrices per condition) had an average of 3.51 x 10⁶ +/-0.8 x 10⁶ cells per matrix. The viability of the recovered cells was 84.5 +/- 2.4%. The *in vitro* production of hFVIII from the gelled matrices was 68,851 +/- 199 mU/24h. Fig. 8 depicts the plasma levels of hFVIII over time after subcutaneous injection of the HSHCM production medium into *Rag*-2 mice. Error bars indicate the standard error of the mean (n = 5 mice per injection volume). Peak expression occurred one day after injection.

### EXAMPLE XII

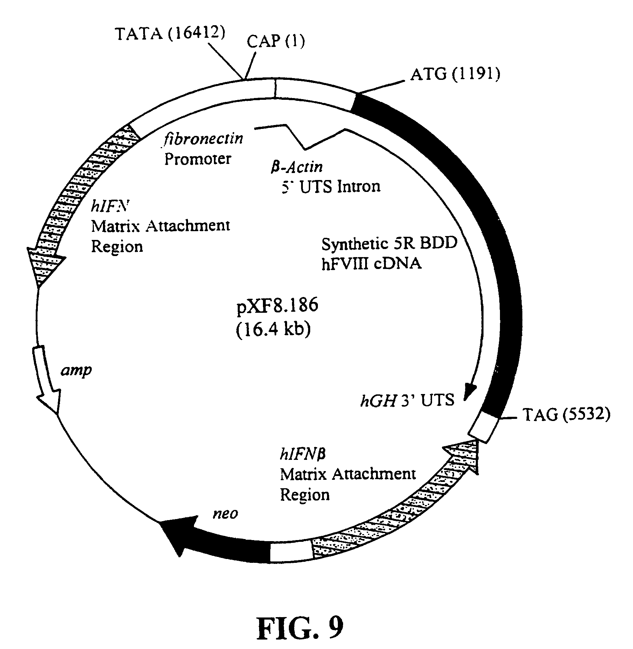

This example describes the intraomental injection into *Nude* mice of rabbit fibroblasts expressing hFVIII, either alone (i.e., without microcarriers) or in combination with heparin-Sepharose® beads coated with growth factors. Heparin-Sepharose® beads were coated with either bFGF (1µg/mL packed beads) or VEGF (2µg/mL packed beads) as described in Example X. Coated beads were distributed into 1.5mL polypropylene microcentrifuge tubes to achieve a volume of 50µL packed beads per tube. Excess PBS was removed from the beads by wicking with sterile absorbent gauze. Cells of RF261 B2-106, a rabbit skin fibroblast clone containing the plasmid pXF8.186 (Fig. 9) and expressing hFVIII at levels between 20,000 - 25,000 mU/24h/10⁶ cells, were harvested from tissue culture flasks by conventional methods. Trypsinized fibroblasts were centrifuged to a pellet (500*xg* for 10 min), washed with Hank's Balanced Salt Solution (HBSS), centrifuged, and resuspended in a volume of PBS to achieve a cell density of approximately 15 x 10⁶ cells/mL. A volume equaling 5 x 10⁶ cells was added either to empty 1.5 mL microcentrifuge tubes or to the tubes containing 50µL of heparin-Sepharose® beads, and the suspensions were centrifuged at 500*xg* for 3 min. The final volume in the tubes containing the beads and cells was adjusted to 100µL by removing PBS. Twelve replicates were prepared for each condition. The tubes containing the cell pellets and cell/bead slurries were placed on ice and transported to the Animal Facility.

For intraomental (IO) injection of cells and beads, *Nude* mice (NIH Swiss *Nude* (nu/nu, Tac:N:NIH(S)-Hfhllnu, Taconic Farms) were anesthetized and prepped as described in Example II. Animals were placed in lateral recumbency and the left flank between the ribs and stifle wiped with an alcohol pad and prepped with Betadine™. A small (0.5 cm to 1.0 cm) incision was made posterior to the ribs and ventral to the spine. The spleen was exposed and gently exteriorized. Using a 20-gauge cannula, cell pellets and cell/bead slurries were injected along the axis of the spleen upon the cranial and medial aspect and within the thin membrane adjacent to the hilar surface of the spleen. The spleen was replaced in the abdominal cavity, and the incision closed.

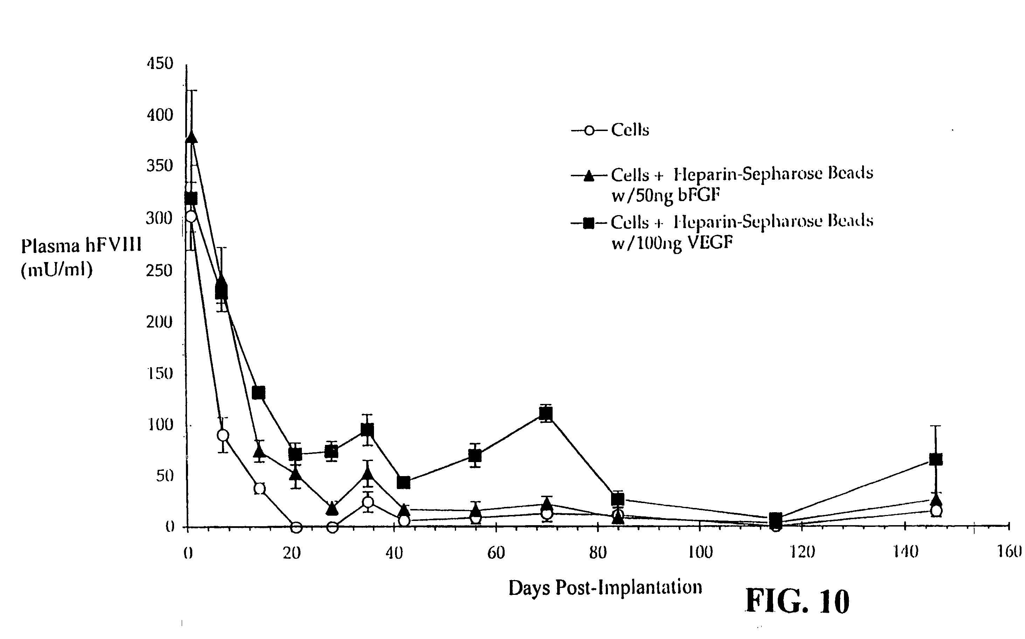

Methods for blood collection and determination of hFVIII were as described in Example II. Fig. 10 shows the plasma levels of hFVIII over time after the injection of cells alone, cells and heparin-Sepharose® beads coated with bFGF (50ng total per implant), and cells and heparin-Sepharose® beads coated with VEGF (100ng total per implant). Error bars indicate the standard error of the mean (n = 12 mice per condition). Peak expression occurred on day 1. The cell/bead/VEGF combination led to significantly higher hFVIII levels between days 28 and 70 compared to the other conditions.

### EXAMPLE XIII

This example describes the intraomental injection into *Nude* mice of rabbit fibroblasts expressing hFVIII, either alone or in combination with heparin-Sepharose® beads coated with growth factors.

The heparin-Sepharose® beads were either uncoated or coated with bFGF (10µg/mL packed beads) or VEGF (2µg/mL packed beads) as described in Example I. The uncoated and growth factor-coated beads were distributed into tubes as described in Example XII. Cells ofRF302 B2-383, a rabbit skin fibroblast clone containing the plasmid pXF8.186 and expressing hFVIII at levels between 20,000-25,000 mU/24h/ 10⁶ cells, were harvested from tissue culture flasks by conventional methods. The cells were processed and distributed into empty microcentrifuge tubes or tubes containing heparin-Sepharose® beads as described in Example VI. Intraomental injection, blood collection, and hFVIII measurement were carried out as described in Example VI.

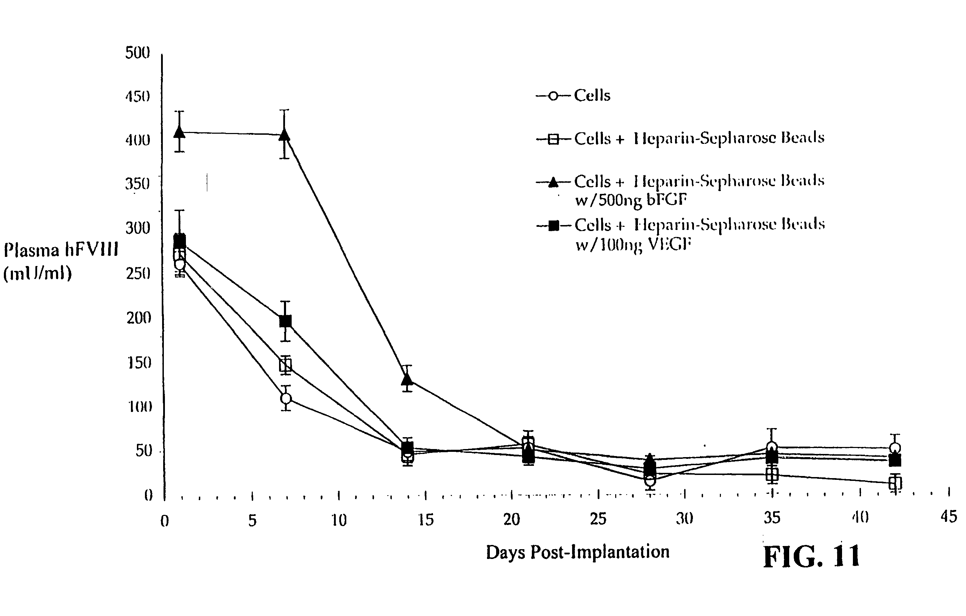

Fig. 11 depicts the plasma levels of hFVIII over time after the injection of cells alone, cells and uncoated heparin-Sepharose® beads, cells and heparin-Sepharose® beads coated with bFGF (500ng total per implant), and cells and heparin-Sephamse® beads coated with VEGF (100ng total per implant). Error bars indicate the standard error of the mean (n = 12 mice per condition). Peak hFVIII expression occurred on day 1, and was significantly greater for the bFGF set compared to all other conditions on days 1, 7, and 14. On day 7, the cell/bead/VEGF combination led to significantly higher hFVIII levels compared to the cells alone and the cell/uncoated bead combination.

### EXAMPLE XIV

This example describes the intraomental injection into *Nude* mice of rabbit fibroblasts expressing hFVIII, either with macroporous collagen microcarriers or with microcarriers in combination with heparin-Sepharose® beads coated with growth factors.

Heparin-Sepharose® beads were either uncoated or coated with bFGF (10µg/mL packed beads) or VEGF (2µg/mL packed beads) as described in Example I. The microcarriers and cells were prepared as follows. Macroporous collagen microcarriers (Cellex Biosciences) were conditioned as described in Example L A slurry of microcaniers (1:1 volume of microcarriers:conditioning medium) was prepared, and 0.1mL aliquots were added to 1.5mL microcentrifuge tubes. These tubes were centrifuged at 500_{*xg*} for 4 min, aspirated, and washed 2x with PBS. The PBS was removed and the tubes were stored on ice. A 0.1mL slurry of heparin-Sepharose® beads (uncoated, coated with bFGF, or coated with VEGF) in PBS at a 1:1 ratio (vol/vol) was added to the tubes containing microcarriers and the bead/microcarrier mixtures were centrifuged at 500_{*xg*} for 4 min. The PBS was removed from each tube and the beadhnicrocarrier pellets were stored on ice. RF302 B2-383 cells were processed as described in Example XII. A volume of cell suspension containing 5 x 10⁶ cells was added to each tube containing either microcarriers alone or microcarriers and heparin-Sepharose® beads, and the tubes were centrifuged at 500_{*xg*} for 4 min. The supernatant was removed from each tube and 10µl of PBS was added. Tubes were placed on ice and transported to the Animal Facility. Intraomental injection, blood collection, and hFVIII measurement were carried out as described in Example XII.

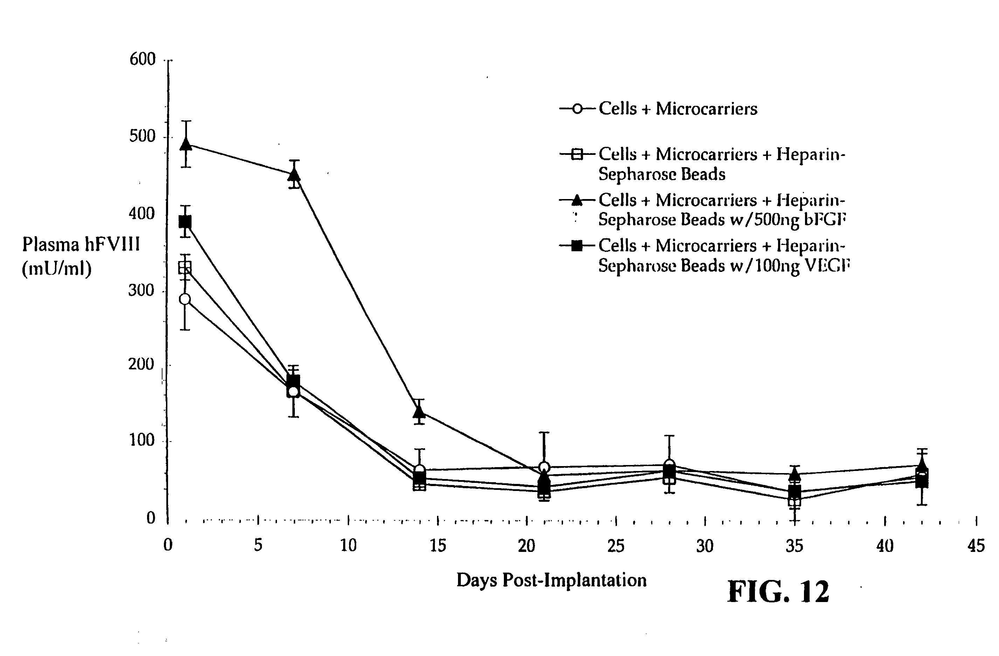

Fig. 12 shows the plasma levels of hFVIII over time after injection of cells and microcarriers, cells and microcarriers and uncoated heparin-Sepharose® beads; cells and microcarriers and beads coated with bFGF (500ng total per implant); and cells and microcarriers and beads coated with VEGF (100ng total per implant). Error bars indicate the standard error of the mean (n = 12 mice per condition). Peak hFVIII expression occurred on day 1, and was significantly greater for the bFGF set compared to the other conditions on days 1, 7, and 14.

### Example XV

Keratinocytes are epidermal cells of the skin, and are normally contiguous with fibroblasts in this tissue. These two cell types depend on one another for the proper nutrients, differentiation signals, and production and maintenance of surrounding extracellular matrix. The addition of keratinocytes to any of the matrices or injectable mixtures described herein that contain fibroblasts can benefit the fibroblasts in a manner similar to what occurs in the skin. The keratinocytes and fibroblasts can be isolated from the same skin biopsy. The keratinocytes can be added simultaneously with the fibroblasts at the time of mixture formation, or they can be seeded onto a contracted matrix containing fibroblasts. The keratinocytes within or on the matrix can be stimulated to differentiate, or not, depending upon the growth medium formulation and culture conditions. For example, an increase in the concentration of calcium ion in the growth medium, and the exposure of one side of the matrix to air, can cause the keratinocytes within the matrix to stratify in a manner similar to what occurs in the skin (Bell *et al., J. Biomech. Eng*. 113:113-119, 1991; Parenteau *et al., J.Cell. Biochem.* 45:245-251, 1991). Once differentiated, the keratinocytes lay down basal lamina consisting of collagen, glycosaminoglycans, and laminin. These basal lamina components as well as extracellular matrix proteins, which can be either synthesized by fibroblasts or included in the production medium, provide a physiological framework that is advantageous to fibroblast survival, e.g., after implantation of the matrix in a subject or after *in vivo* formation of matrices from mixtures introduced into a subject.

### Example XVI

Endothelial cells form the lumen of blood vessels and capillaries. Vessels are formed from single endothelial cells that divide and differentiate to form contiguous tubes. This differentiation process can be stimulated in the presence of essential environmental factors. These factors include collagen, extracellular matrix proteins produced by cells such as fibroblasts and/or keratinocytes, and growth factors (e.g., basic fibroblast growth factor or vascular endothelial growth factor). Implanted matrices, or matrices formed *in vivo* after introduction of a mixture of the invention into a subject, can provide the proper environment for endothelial tube formation if endothelial cells are added to the matrix production medium or mixtures, respectively, along with fibroblasts. The addition ofkeratinocytes can help induce differentiation of the endothelial cells through secretion of factors such as vascular endothelial growth factor as well as production of a basal lamina. The formation of endothelial tubes within the matrix can accelerate the vascularization of the matrix after implantation by inosculation of the pre-existing endothelial tubes with the growing blood vessels of the host (Black et al., FASEB J. 12:1331-1340, 1998; Black et al., Cell Biol. Toxicol. 15(2):81-90, 1999). Endothelial cells can also be beneficial by releasing factors that promote neovascularization.

### EXAMPLE XVII

The mixtures of the invention would be prepared for introduction into humans as follows:

The desired cells, typically stably transfected autologous cells derived from the patient, are harvested from tissue culture dishes and processed for the production of HCMM or PCHCMM by any of the methods described above. The dosage for a given patient (i.e., the physiologically effective quantity of therapeutic product produced by the matrix) can be varied by introducing a larger or smaller volume of the mixture into the patient, and/or using cells which express a different level of the product per cell when making the mixture. The quantity of the therapeutic product produced in the patient can also be varied by exposing the cells in the mixture to a pharmacologic or physiologic signal which alters expression of the therapeutic gene. For example, if the therapeutic gene is under the control of a glucocorticoid-responsive promoter, then *in vivo* exposure of the cells to a drug such as dexamethasone (by administering the drug to the patient in a manner that ensures the drug reaches the implant) will alter expression of the therapeutic gene. Other pharmacologically responsive control elements are described in Clackson, Gene Therapy 7:120-125, 2000, which is incorporated herein by reference in its entirety. Such elements include those regulated by the antibiotic tetracycline or its analogs (e.g., doxycycline), the insect steroid ecdysone or its analogs, the antiprogestin mifepristone (RU486), and chemical "dimerizers" such as rapamycin and its analogs ("rapalogs").

The mixtures can contain any of the microcarriers, collagens (or alternatives), agents (optionally attached to solid substrates), and cells producing agents described herein. They can also optionally contain keratinocytes and/or endothelial cells. Microcarriers loaded with any of the cells recited herein can be frozen and then thawed prior to use in the injectable mixtures.

The mixture can be introduced at a variety of sites, including but not limited to subcutaneous, intraperitoneal, intrasplenic, intraomental, inguinal, intrathecal, intraventricular, and intramuscular sites, as well as within lymph nodes or within adipose tissue. The mixture can be introduced using, for example, a hypodermic syringe (plastic or glass) optionally capped with a needle or catheter. The diameter of the needle or catheter is preferably larger than the diameter of microcarriers (e.g., greater than 0.5 mm) to minimize damage to the suspended cells and microcarriers. Delivery to an appropriate internal site can be facilitated by the use of a laparoscope. Under certain circumstances, it may be desirable to make a surgical incision, introduce the mixture and then close the incision.

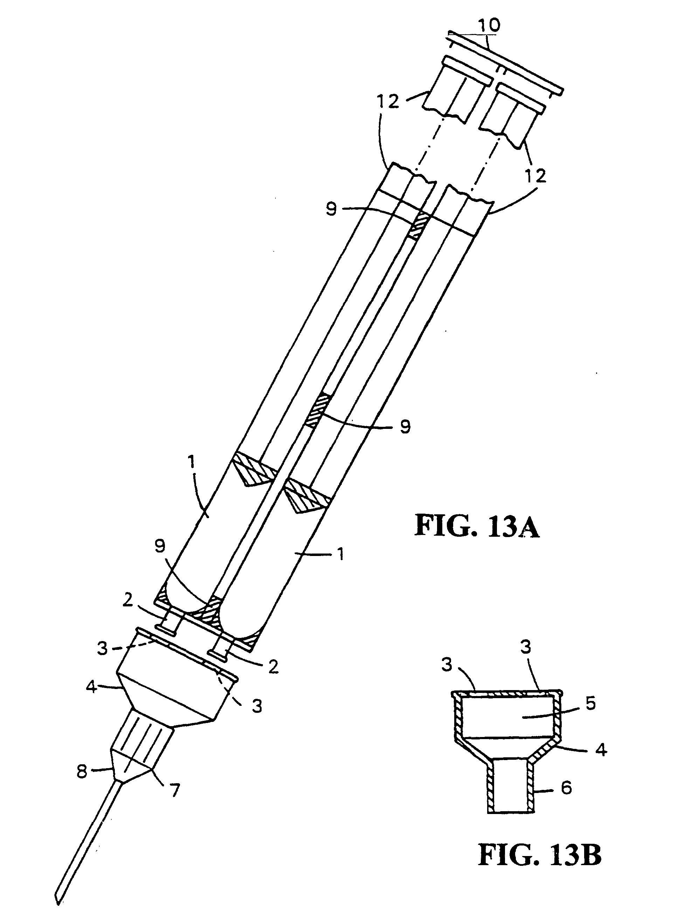

The mixtures of the invention can be simultaneously formed and introduced into a patient (or any other animal). An appropriate apparatus can be constructed for this purpose. An example of such an apparatus is shown in Fig. 13A. The apparatus can contain two or more vessels **1**, all of which connect via outlets **2** to inlets **3** in an adapter piece **4**. Fig. 13B shows a cross-sectional view of such an adapter piece **4**. The inlets **3** in the adapter piece **4** are in fluid communication, preferably in a liquid-tight fashion, with the outlets **2** from the two or more vessels. Alternatively, the adapter piece **4** can contain a single connector that is in fluid communication, preferably in a liquid-tight fashion, with all the outlets **2** of the vessels **1**. The adapter piece also contains a mixing chamber **5** and an outlet **6** that is in fluid communication, preferably in a liquid-tight fashion, with the single inlet in the hub **7** of a syringe needle **8**. In this way, when the contents of the vessels are caused (by simultaneously applying pressure to pistons **12**) to flow from the outlets **2** of the vessels **1**, they enter the mixing chamber **5** in the adapter piece **4** where they mix, and the resulting mixture flows out of the outlet **5** in the adapter piece **4** and into the svrinae needle **8**. The mixture is then forced into the needle portion **13** of the syringe needle and, hence, into a tissue or body space of an animal into which the needle has been inserted. The vessels are physically joined to each other by solid connectors **9**. A "pressure plate" **10** connecting the tops of pistons **12** is included in order to facilitate simultaneous extrusion of the contents of the vessels.

In another embodiment, instead of an adapter piece, the hub section of the syringe needle can be adapted to have separate inlets that are in fluid communication, preferably in a liquid-tight manner, with the individual outlets in the vessels. When the contents of all the vessels are caused to flow out of the outlets of the vessels (e.g., by simultaneously applying pressure to the plungers, where the vessels are syringes), they enter the hub of the syringe needle via the appropriate inlets in the hub and mix in the small chamber formed within the hub before entering the needle portion of the apparatus. As used herein, a "delivery means" can be: (a) a syringe needle adapted as described, (b) the above described adapter piece **4** and the syringe needle **8**, (c) a catheter, (d) a cannula, (e) a pipette or any tube-shaped object, appropriately adapted, that will function as described.

In an apparatus with two vessels, one of the vessels can contain, for example, cells, microcarriers, and agents in a nutrient medium, and the other can contain a collagen solution. In a three-vessel apparatus, one vessel can contain, for example, cells, microcarriers, and agents in nutrient medium; the second vessel can contain a collagen solution at an acid pH; and the third can contain medium or diluent at an alkaline pH, such that when the contents of all three vessels mix, an aqueous suspension at a pH of about 7.2 to about 7.8, preferably about 7.4 to about 7.8, is formed. It is understood, however, that the various components of the hybrid matrix mixtures of the invention can be distributed among the two or three vessels of the device in any convenient combination. While in using such an apparatus, introduction of the mixture into the animal occurs shortly after formation of the mixture from its components, for the purposes of the invention, the two events can be said to occur simultaneously.

The invention includes a vessel containing any of the mixtures disclosed herein (see "Summary of the Invention"). Such a vessel can be included together with shipping material (e.g., a shipping container), in a kit. The feasibility of shipping the injectable mixtures in a single vessel containing all of the components was evaluated. Two studies with injectable HSHCM fluid mixtures were performed. For both, the heparin-Sepharose® bead component was coated with bFGF at a concentration of 50 _{µ}g/mL packed beads. A volume of 10mL of complete production medium, prepared as outlined in Table 9, was added to each of three 10cc plastic syringes, which were then capped and stored at 4°C for 24h. The syringes were removed from the 4°C environment, capped with 16G needles, and the contents injected into 100mm petri dishes. The dishes were placed in an incubator at a temperature of 37°C, humidity of 95% to 98%, and CO₂ level of 5%. After 1h, the dishes were removed and fed by addition of 20mL of DMEM without phenol red, and returned to the incubator. The decrease in diameter of the solidified matrix, relative to the diameter of the liquid mixture ("reduction in diameter"), cell number, and cell viability were assessed after 24h of incubation at 37°C. The "reduction in diameter" was determined by placing a ruler beneath each dish, measuring the matrix diameter in centimeters, and subtracting the matrix diameter from the diameter of the 100mm dish. The difference in diameter was then divided by the diameter of the 100mm dish and expressed as a percentage. Cell number and viability were assessed as described above. Table 10 summarizes the results of the two storage experiments. Experiments #1 and #2 differed in the number of cells incorporated per mL production medium as described (n=3 syringes per study).

**TABLE 10**

| Injectable HSHCM Storage Study | | | | | | |
|---|---|---|---|---|---|---|
| Exp# | #Cells/ 10mL (x 10⁶) | Time of 4°C Storage | Time of 37°C Incubation | # Cells Recovered (x 10⁶) | % Viability | % Reduction in Diameter |
| 1 | 44.7 | 24h | 24h | 39.5+/-4.1 | 92+/-1.5 | 26.9+/-3.9 |
| 2 | 91.0 | 24h | 24h | 93.4+/-5.1 | 94+/-3.7 | 35.4+/-0.7 |

Alternatively, since a neutralized collagen solution will gel more rapidly than an acidic collagen solution, it could be advantageous to separate the components of the injectable material in a kit for the purpose of shipping the material to an offsite location. For example, a kit could contain three vessels (e.g., tubes, syringes, bottles, or vials), the contents of which are combined immediately prior to introduction into the patient (i.e., before the mixture has solidified to such an extent that it does not flow readily from the syringe, cannula, or whatever means is used to deliver the suspension). The components could, for example, be separated in the following manner: vessel 1 can contain cells + microcarriers, and, optionally, heparin-Sepharose® beads in a slurry; vessel 2 can contain a collagen solution at acidic pH (about 2.0 - about 4.0); and vessel 3 can contain concentrated medium (e.g., nutrient medium or other diluent) without serum and at alkaline pH (about 9.0 - about 12.0). The pH of the concentrated medium would be selected to ensure neutralization of the collagen solution to a physiological pH (e.g., pH 7.4) when the medium and collagen solutions are combined. Immediately prior to injection into the patient, the contents of vessel 3 (medium) would be mixed with the contents of vessel 2 (collagen solution). The combined medium + collagen, which will at this time be neutralized to physiological pH, would then be added to the cell/microcarrier/heparin-Sepharose® bead slurry and mixed thoroughly to form the final mixture. This final mixture would then be injected into the patient, optionally using a hypodermic syringe or other liquid-dispensing apparatus supplied with the kit.

Alternatively, a kit could contain two vessels, with the first vessel containing the same components as vessel 1 in the kit described above. The second vessel could contain a collagen solution in which the collagen is dissolved, for example, in nutrient medium and which is at an acidic pH. Immediately prior to injection, the pH of the collagen solution is adjusted to about 7.2 to about 7.4, preferably about 7.4 to about 7.8, using an alkaline solution (e.g., a NaOH solution). An aliquot of such an alkaline solution could optionally be included in a third vessel in the kit.

Instead of providing the collagen solution at an acidic pH, it can be provided at a pH of about 7.2 to about 7.8, preferably about 7.4 to about 7.8, as long as it is kept cold (e.g., below 8°C) until immediately prior to mixing with the other components and introduction into the patient.

The kits of the invention can optionally include labels or inserts with instructions for use of the kit, e.g., instructions on how to combine the contents of the vessels and administer the resulting mixture to a subject. Keeping the mixture cold prevents gelling of the collagen.

### Other Embodiments

The matrices and mixtures of the invention are appropriate for delivery of a wide range of cellular products, including not only hGH and Factor VIII, but also Factor IX, erythropoietin (EPO), albumin, hemoglobin, alpha-1 antitrypsin, calcitonin, glucocerebrosidase, low density lipoprotein (LDL) receptor, IL-2 receptor, globin, immunoglobulin, catalytic antibodies, the interleukins, insulin, insulin-like growth factor I (IGF-1), insulinotropin, parathyroid hormone (PTH), leptin, an IFN (e.g., IFN_{α}-, IFN-_{β}, or IFN-_{γ}), the nerve growth factors, basic fibroblast growth factor (bFGF), acidic FGF (aFGF), epidermal growth. factor (EGF), endothelial cell growth factor, platelet derived growth factor (PDGF), transforming growth factors, endothelial cell stimulating angiogenesis factor (ESAF), angiogenin, tissue plasminogen activator (t-PA), granulocyte colony stimulating factor (G-CSF), granulocyte-macrophage colony stimulating factor (GM-CSF), _{α}-galactosidase, and FSH. For example, the cells in the mixtures or embedded in the matrices can be pancreatic beta cells which naturally secrete insulin in response to a rise in blood glucose, and which therefore can supplement an inadequate insulin response in a diabetic or pre-diabetic patient. Alternatively, they can be any type of cell genetically engineered to express and secrete high levels of a needed polypeptide, such as a clotting factor, within the patient. Such a construct can be under the control of a constitutively activated promoter, or of an appropriately physiologically or pharmacologically regulated promoter.

The collagen gel portion of the mixture or matrix can consist entirely of collagen, or can contain other components in addition to collagen: e.g., agarose; alginate; a glycosaminoglycan such as hyaluronic acid, heparin sulfate, dermatan sulfate, chondroitin sulfate; a sulfated proteoglycan; fibronectin; laminin; elastin; fibrin; tenascin; minced adipose tissue; minced omental tissue; methyl cellulose; or gelatin. Such components (particularly those which are found in the extracellular matrix of animal tissues) contribute to the structural stability of the hybrid matrices of the invention and matrices obtained by *in vivo* solidification of mixtures of the invention, and/or provide additional attachment capacity for the cells in the matrices and the host tissue at the site of matrix implantation. They would be mixed into the matrix mixture prior to introduction into an animal or prior to gelling *in vitro*.

Other potential additives include cytokines and/or growth factors which are useful for optimizing maintenance of the cells or promoting beneficial interaction with host tissue (e.g., vascularization), including bFGF, aFGF, endothelial cell growth factor, PDGF, endothelial cell stimulating angiogenesis factor (ESAF), leukotriene C₄, prostaglandins (e.g., PGE₁, PGE₂), IGF-1, GCSF, angiogenin, TGF-_{α}, TGF-_{β}, ascorbic acid, EGF, oncostatin M, VEGF, VEGF-A, VEGF-B, VEGF-C, and VEGF-D. These additives can be incorporated into the matrix by mixing them with the collagen solution prior to gelling or introduction into an animal, by introducing them into the interstices of the microcarriers, or by including them in the medium which bathes the matrices. They can also be bound to or encapsulated within separate solid substrates (e.g., heparin-coated agarose® beads or PLGA microcapsules) included in the matrices or mixtures. Alternatively, the cells can be genetically engineered to express the desired product. For example, the cells of the matrix can be cotransfected with a DNA encoding an angiogenesis factor and a DNA encoding a second, therapeutic protein, or with a single DNA construct encoding both types of proteins linked to suitable expression control sequences.

Ascorbic acid promotes the production of mature collagen by fibroblasts by promoting the proper post-translational modification of procollagen. The production of collagen by fibroblasts embedded within the matrix or mixture will increase the physical strength of the matrix as well as provide a physiological architecture that can increase cell survival in an animal.

The collagen used in the matrices and mixtures can be any suitable type (e.g., type I-XI), or a mixture of any two or more. Fibers can be placed in the mold prior to gelling of the collagen, so that they become an integral part of the matrix and contribute to the sturdiness and handling convenience of the matrix. If fibers are added, they would be made principally of collagen (e.g., cat gut) or a non-collagenous material such as nylon, dacron, polytetrafluoroethylene (Gore-Tex™ or Teflon™), polyglycolic acid, polylactic/polyglycolic acid mixture (Vicryl™), polystyrene, polyvinylchloride co-polymer, cellulose (e.g., cotton or linen), polyester, rayon, or silk. The fibers in the matrices can be woven into a mesh or cloth, or used as individual threads.

Instead of the type I collagen microcarriers described in the above examples, one could utilize microcarriers consisting primarily of another type of collagen, polystyrene, dextran (e.g., Cytodex™, Pharmacia), polyacrylamide, cellulose, calcium alginate, latex, polysulfone, glass (coated with a substance such as collagen which promotes cellular adherence), gelatin, or combinations of collagen with any of the above. Such microcarriers are available commercially or can be made by standard methods, then sterilized for use in the matrices and mixtures of the invention.

Other embodiments are within the following claims.

## Claims

1. A composition comprising a body of matrix material comprising insoluble collagen fibrils, there being embedded within the body of matrix material
(a) a population of cultured vertebrate cells genetically engineered to express a polypeptide;
(b) a plurality of microcarriers; and
(c) an agent bound to a solid substrate embedded in the body of the matrix material, wherein the agent is selected from the group consisting of a factor which promotes vascularization, a cytokine, a growth factor and ascorbic acid.

2. The composition of claim 1, wherein the solid substrate comprises (a) heparin or (b) heparan sulfate proteoglycan.

3. The composition of claim 1, wherein the solid substrate comprises agarose with heparin or heparan sulfate proteoglycan bound thereto.

4. The composition of claim 3, wherein the solid substrate further comprises calcium alginate.

5. The composition of claim 1, wherein the solid substrate further comprises calcium alginate.

6. The composition of claim 1, wherein the solid substrate comprises a substance selected from the group consisting of collagen, gelatin, ethylene-vinyl acetate, polylactide/glycolic acid co-polymer, fibrin, sucrose octasulfate, dextran, polyethylene glycol, an alginate, polyacrylamide, cellulose, latex, and polyhydroxyethylmethacrylate.

7. The composition of claim 6, wherein heparin or heparan sulfate proteoglycan is bound to the substrate.

8. The composition of claim 1, wherein the composition further comprises a second agent selected from the group consisting of a factor which promotes vascularization, a cytokine, and a growth factor.

9. The composition of claim 1, wherein the solid substrate is in the form of beads.

10. The composition of claim 1, wherein the solid substrate is in the form of threads.

11. The composition of claim 1, wherein the agent is basic fibroblast growth factor (bFGF).

12. The composition of claim 1, wherein the agent is selected from the group consisting of acidic fibroblast growth factor (aFGF), endothelial cell growth factor, platelet-derived growth factor (PDGF), endothelial cell stimulating angiogenesis factor (ESAF), leukotriene C₄, a prostaglandin, insulin-like growth factor 1 (IGF-1), granulocyte colony stimulating factor (G-CSF), angiogenin, transforming growth factor-α (TGF-α), transforming growth factor-β (TGF-β), ascorbic acid, epidermal growth factor (EGF), and oncostatin M.

13. The composition of claim 1, wherein the agent is selected from the group consisting of vascular endothelial growth factor (VEGF), VEGF-A, VEGF-B, VEGF-C, and VEGF-D.

14. The composition of claim 1, wherein the agent is angiopoietin 1, 2, 3, or 4.

15. The composition of claim 1, further comprising cells secreting the agent.

16. The composition of claim 1, wherein the cultured vertebrate cells are selected from the group consisting of adipocytes, astrocytes, cardiac muscle cells, chondrocytes, endothelial cells, epithelial cells, fibroblasts, gangliocytes, glandular cells, glial cells, hematopoietic cells, hepatocytes, keratinocytes, myoblasts, neural cells, osteoblasts, pancreatic beta cells, renal cells, smooth muscle cells, striated muscle cells, and precursors of any of the above.

17. The composition of claim 1, wherein the cultured vertebrate cells are human cells.

18. The composition of claim 1, wherein the polypeptide is selected from the group consisting of enzymes, hormones, cytokines, colony stimulating factors, vaccine antigens, antibodies, clotting factors, regulatory proteins, transcription factors, receptors, and structural proteins.

19. The composition of claim 1, wherein the polypeptide is Factor VIII.

20. The composition of claim 1, wherein the polypeptide is human growth hormone.

21. The composition of claim 1, wherein the polypeptide is Factor IX.

22. The composition of claim 1, wherein the polypeptide is erythropoietin.

23. The composition of claim 1, wherein the polypeptide is selected from the group consisting of VEGF, VEGF-A, VEGF-B, VEGF-C, and VEGF-D.

24. The composition of claim 1, wherein the polypeptide is insulinotropin.

25. The composition of claim 1, wherein the polypeptide is selected from the group consisting of alpha-1 antitrypsin, calcitonin, glucocerebrosidase, low density lipoprotein (LDL) receptor, IL-2 receptor, globin, immunoglobulin, catalytic antibodies, the interleukins, insulin, insulin-like growth factor 1 (IGF-1), parathyroid hormone (PTH), leptin, the nerve growth factors, basic fibroblast growth factor (bFGF), acidic FGF (aFGF), epidermal growth factor (EGF), endothelial cell growth factor, platelet derived growth factor (PDGF), transforming growth factors, endothelial cell stimulating angiogenesis factor (ESAF), angiogenin, tissue plasminogen activator (t-PA), granulocyte colony stimulating factor (G-CSF), and granulocyte-macrophage colony stimulating factor (GM-CSF).

26. The composition of claim 1, wherein the microcarriers are beads of type I collagen.

27. The composition of claim 26, wherein the microcarriers are porous.

28. The composition of claim 1, wherein the microcarriers are beads of porous gelatin.

29. The composition of claim 1, wherein the majority of the microcarriers have an approximately spherical shape and have a diameter between approximately 0.1 and approximately 2 mm.

30. The composition of claim 1, wherein the matrix material additionally comprises a substance selected from the group consisting of a second type of collagen, agarose, alginate, fibronectin, laminin, hyaluronic acid, heparan sulfate, dermatan sulfate, sulfated proteoglycans, fibrin, elastin, and tenascin.

31. The composition of claim 1, additionally comprising noncollagen fibers dispersed within the body of matrix material.

32. The composition of claim 31, wherein the noncollagen fibers comprise a material selected from the group consisting of nylon, dacron, polytetrafluoroethylene, polyglycolic acid, polylactic/polyglycolic acid copolymer, polystyrene, polyvinylchloride, cat gut, cotton, linen, polyester, and silk.

33. The composition of claim 1, configured to be implanted into a patient.

34. The composition of claim 33, wherein the cultured vertebrate cells are derived from one or more cells removed from the patient.

35. The composition of claim 34, wherein the cultured vertebrate cells consist of a clonal population.

36. The composition of claim 1, wherein each of the plurality of microcarriers consists primarily of one or more substances selected from the group consisting of collagen, polystyrene, dextran, polyacrylamide, cellulose, calcium alginate, latex, polysulfone, and glass.

37. The composition of claim 1, wherein the cultured vertebrate cells are fibroblasts.

38. The composition of claim 1, wherein the microcarriers have an approximately spherical shape.

39. The composition of claim 37, further comprising keratinocytes.

40. The composition of claim 39, wherein the keratinocytes and the fibroblasts are obtained from the same individual.

41. The composition of claim 39, further comprising one or more keratinocyte differentiation factors.

42. The composition of claim 41, wherein the keratinocyte differentiation factor comprises calcium ions at a concentration of 1.5-2 mM.

43. The composition of claim 1, further comprising endothelial cells.

44. The composition of claim 37, further comprising endothelial cells.

45. The composition of claim 44, wherein the endothelial cells and the fibroblasts are obtained from the same individual.

46. The composition of claim 1, wherein the polypeptide is selected from the group consisting of interferon-α (IFN-α), interferon-β (IFN-β), interferon-γ (IFN-γ), follicle stimulating hormone (FSH), α-galactosidase, β-gluceramidase, α-iduronidase, iduronate 2-sulfatase, glucosamine-N-sulfatase, α-N-acetylglucosaminidase, acetylcoenzyme A:α-glucosaminide-N-acetyltransferase, N-acetylglucosamine-6-sulfatase, β-galactosidase, N-acetylgalactosamine-6-sulfatase, and β-glucuronidase.

47. The composition of claim 1, wherein the matrix material additionally comprises a substance selected from the group consisting of heparin, cellulose, starch and dextran.

48. The composition of claim 1, further comprising collagen.

49. A composition according to any of claims 1 to 47, for the treatment of a patient in need of the polypeptide.

50. A method of making a composition as defined in claim 1, the method comprising
forming a mixture comprising:
(a) a plurality of cultured vertebrate cells genetically engineered to express a polypeptide;
(b) a plurality of microcarriers;
(c) a solution comprising soluble collagen; and
(d) an agent bound to a solid substrate, wherein the agent is selected from the group consisting of a factor that promotes vascularization, a cytokine, a growth factor, and ascorbic acid;
subjecting the soluble collagen in the mixture to conditions effective to form a gel; and
exposing the gel to culture conditions which cause the gel to contract, thereby forming the body of the composition.

51. The method of claim 50, wherein the solution is an acidic aqueous solution of soluble collagen, and gelation is accomplished by raising the pH of the solution.

52. The method of claim 50, wherein the gelation step takes place in a mold, so that, prior to the contracting step, the gel is in the shape of the mold.

53. The method of claim 50, wherein the cultured vertebrate cells are cultured in the presence of the microcarriers prior to being mixed with the solution.

54. The method of claim 50, wherein the gel is formed in a flat-bottomed mold filled with the mixture to a depth of about 0.18 mm.

55. The method of claim 48, wherein the gel is formed in a flat-bottomed cylindrical mold with an internal radius (r); the mixture has a volume (V); and when r is expressed in cm and V is expressed in ml, r²/V is about 1.8.

56. The method of claim 50, further comprising adding keratinocytes to the body of the composition after the gel has contracted.

57. The use of a population of cultured vertebrate cells genetically engineered to express a polypeptide, in the preparation of a composition as defined in claim 1 for the treatment of a patient in need thereof wherein the cultured vertebrate cells secrete the polypeptide.

58. The use of claim 57, wherein the cultured vertebrate cells are derived from one or more cells removed from a patient, and have been genetically engineered *in vitro* to express and secrete the polypeptide.

59. The use of claim 57, wherein the composition is configured to be implanted at a subcutaneous site in a patient.

60. The use of claim 57, wherein the composition is configured to be implanted at an intraperitoneal, sub-renal capsular, inguinal, intramuscular, intraventricular, intraomental, or intrathecal site in a patient.

61. The use of claim 57 wherein the composition is for treatment of a wound, wherein the polypeptide is one which promotes wound healing, and the composition is configured to be implanted at the site of a preexisting wound of a patient.

62. A mixture comprising, suspended in an aqueous collagen solution,
(a) a plurality of cultured vertebrate cells genetically engineered to express a polypeptide,
(b) a plurality of microcarriers, and
(c) at least one agent selected from the group consisting of a factor which promotes vascularization, a cytokine, a growth factor, and ascorbic acid, wherein the at least one agent is bound to a solid substrate suspended in the collagen solution,
wherein there is sufficient collagen in the mixture to allow the mixture to gel at a pH above 5.

63. A vessel comprising a mixture comprising, suspended in an aqueous collagen solution,
(a) a population of cultured vertebrate cells genetically engineered to express a polypeptide;
(b) a plurality of microcarriers; and
(c) an agent associated with a solid substrate, wherein the agent is selected from the group consisting of a factor which promotes vascularization, a cytokine, a growth factor, and ascorbic acid,
wherein there is sufficient collagen in the mixture to allow the mixture to gel at a pH above 5.

## Patentansprüche

1. Zusammensetzung, umfassend einen Matrixmaterialkörper, der unlösliche Kollagenfibrillen umfasst, wobei in dem Matrixmaterialkörper Folgendes eingebettet ist:
(a) eine Population kultivierter Vertebraten-Zellen, die zur Expression eines Polypeptids genetisch manipuliert sind;
(b) eine Vielzahl von Mikroträgern; und
(c) ein Mittel, das an ein festes Substrat gebunden ist, eingebettet in den Körper des Matrixmaterials, worin das Mittel aus der Gruppe ausgewählt ist, bestehend aus einem Faktor, der die Vaskularisation fördert, einem Cytokin, einem Wachstumsfaktor und Ascorbinsäure.

2. Zusammensetzung nach Anspruch 1, worin das feste Substrat (a) Heparin oder (b) Heparansulfat-Proteoglycan umfasst.

3. Zusammensetzung nach Anspruch 1, worin das feste Substrat Agarose mit Heparin oder Heparansulfat-Proteoglycan daran gebunden umfasst.

4. Zusammensetzung nach Anspruch 3, worin das feste Substrat weiter Calciumalginat umfasst.

5. Zusammensetzung nach Anspruch 1, worin das feste Substrat weiter Calciumalginat umfasst.

6. Zusammensetzung nach Anspruch 1, worin das feste Substrat eine Substanz umfasst, die aus der Gruppe ausgewählt ist, bestehend aus Kollagen, Gelatine, Ethylen-Vinylacetat, Polylactid/Glycolsäure-Copolymer, Fibrin, Saccharose-Octasulfat, Dextran, Polyethylenglycol, einem Alginat, Polyacrylamid, Cellulose, Latex und Polyhydroxyethylmethacrylat.

7. Zusammensetzung nach Anspruch 6, worin Heparin oder Heparansulfat-Proteoglycan an das Substrat gebunden ist.

8. Zusammensetzung nach Anspruch 1, worin die Zusammensetzung weiter ein zweites Mittel umfasst, das aus der Gruppe ausgewählt ist, bestehend aus einem Faktor, der die Vaskularisation fördert, einem Cytokin und einem Wachstumsfaktor.

9. Zusammensetzung nach Anspruch 1, worin das feste Substrat in der Form von Perlen vorliegt.

10. Zusammensetzung nach Anspruch 1, worin das feste Substrat in der Form von Fäden vorliegt.

11. Zusammensetzung nach Anspruch 1, worin das Mittel einen basischen Fibroblastenwachstumsfaktor (bFGF) darstellt.

12. Zusammensetzung nach Anspruch 1, worin das Mittel aus der Gruppe ausgewählt ist, bestehend aus einem sauren Fibroblastenwachstumsfaktor (aFGF), Endothelzellenwachstumsfaktor, Platelet-derived Growth Factor (PDGF), Endothelzellen stimulierendem Angiogenesefaktor (ESAF), Leukotrien C₄, einem Prostaglandin, einem insulinartigen Wachstumsfaktor 1 (IGF-1), Granulozytenkolonien-stimulierendern Faktor (G-CSF), Angiogenin, transformierendem Wachstumsfaktor-α (TGF-α), transformierendem Wachstumsfaktor-β (TGF-β), Ascorbinsäure, epidermalem Wachstumsfaktor (EGF) und Oncostatin M.

13. Zusammensetzung nach Anspruch 1, worin das Mittel aus der Gruppe ausgewählt ist, bestehend aus vaskulärem endothelialem Wachstumsfaktor (VEGF), VEGF-A, VEGF-B, VEGF-C und VEGF-D.

14. Zusammensetzung nach Anspruch 1, worin das Mittel Angiopoietin 1, 2, 3 oder 4 darstellt.

15. Zusammensetzung nach Anspruch 1, die weiter Zellen umfasst, die das Mittel absondern.

16. Zusammensetzung nach Anspruch 1, worin die kultivierten Vertebraten-Zellen aus der Gruppe ausgewählt sind, bestehend aus Adipozyten, Astrozyten, Herzmuskelzellen, Chondrozyten, Endothelzellen, Epithelzellen, Fibroblasten, Gangliozyten, Drüsenzellen, Gliazellen, hämatopoietischen Zellen, Hepatozyten, Keratinozyten, Myoblasten, Neuralzellen, Osteoblasten, Pankreas-β-Zellen, Nierenzellen, glatten Muskelzellen, gestreiften Muskelzellen und Präkursoren von jedwedem des Vorstehenden.

17. Zusammensetzung nach Anspruch 1, worin die kultivierten Vertebraten-Zellen humane Zellen darstellen.

18. Zusammensetzung nach Anspruch 1, worin das Polypeptid aus der Gruppe ausgewählt ist, bestehend aus Enzymen, Hormonen, Cytokinen, kolonienstimulierenden Faktoren, Vakzin-Antigenen, Antikörpern, Gerinnungsfaktoren, Regulationsproteinen, Transkriptionsfaktoren, Rezeptoren und Strukturproteinen.

19. Zusammensetzung nach Anspruch 1, worin das Polypeptid Faktor VIII darstellt.

20. Zusammensetzung nach Anspruch 1, worin das Polypeptid humanes Wachstumshormon darstellt.

21. Zusammensetzung nach Anspruch 1, worin das Polypeptid Faktor IX darstellt.

22. Zusammensetzung nach Anspruch 1, worin das Polypeptid Erythropoietin darstellt.

23. Zusammensetzung nach Anspruch 1, worin das Polypeptid aus der Gruppe ausgewählt ist, bestehend aus VEGF, VEGF-A, VEGF-B, VEGF-C und VEGF-D.

24. Zusammensetzung nach Anspruch 1, worin das Polypeptid Insulinotropin darstellt.

25. Zusammensetzung nach Anspruch 1, worin das Polypeptid aus der Gruppe ausgewählt ist, bestehend aus Alpha-1-Antitrypsin, Calcitonin, Glucocerebrosidase, einem Lipoprotein-Rezeptor niedriger Dichte (LDL), IL-2-Rezeptor, Globin, Immunglobulin, katalytischen Antikörpern, den Interleukinen, Insulin, insulinartigem Wachstumsfaktor 1 (IGF-1), Parathormon (PTH), Leptin, den Nervenwachstumsfaktoren, basischem Fibroblasten-Wachstumsfaktor (bFGF), saurem FGF (aFGF), epidermalem Wachstumfaktor (EGF), Endothelzellen-Wachstumsfaktor, Platelet-derived Growth Factor (PDGF), transformierenden Wachstumsfaktoren, Endothelzellen stimulierendem Angiogenesefaktor (ESAF), Angiogenin, Gewebsplasminogenaktivator (t-PA), Granulozytenkolonien-stimulierendem Faktor (G-CSF) und Granulozyten-Makrophagenkolonien-stimulierendem Faktor (GM-CSF).

26. Zusammensetzung nach Anspruch 1, worin die Mikroträger Perlen des Typ-1-Kollagens darstellen.

27. Zusammensetzung nach Anspruch 26, worin die Mikroträger porös sind.

28. Zusammensetzung nach Anspruch 1, worin die Mikroträger Perlen aus poröser Gelatine darstellen.

29. Zusammensetzung nach Anspruch 1, worin die Mehrzahl der Mikroträger eine annähernde sphärische Gestalt aufweisen und einen Durchmesser zwischen ca. 0,1 und ca. 2 mm aufweisen.

30. Zusammensetzung nach Anspruch 1, worin das Matrixmaterial zusätzlich eine Substanz umfasst, die aus der Gruppe ausgewählt ist, bestehend aus einem Typ-2-Kollagen, Agarose, Alginat, Fibronectin, Laminin, Hyaluronsäure, Heparansulfat, Dermatansulfat, sulfatierten Proteoglycanen, Fibrin, Elastin und Tenascin.

31. Zusammensetzung nach Anspruch 1, die zusätzlich nichtkollagene Fasern in dem Matrixmaterialkörper dispergiert umfasst.

32. Zusammensetzung nach Anspruch 31, worin die nichtkollagenen Fasern ein Material umfassen, das aus der Gruppe ausgewählt ist, bestehend aus Nylon, Dacron, Polytetrafluorethylen, Polyglycolsäure, Polymilchsäure/Polyglycolsäure-Copolymer, Polystyren, Polyvinylchlorid, Katgut, Baumwolle, Leinen, Polyester und Seide.

33. Zusammensetzung nach Anspruch 1, die zur Implantation in einen Patienten konfiguriert ist.

34. Zusammensetzung nach Anspruch 33, worin sich die kultivierten Vertebraten-Zellen von einer oder mehr aus dem Patienten entfernten Zelle(n) herleiten.

35. Zusammensetzung nach Anspruch 34, worin die kultivierten Vertebraten-Zellen aus einer klonalen Population bestehen.

36. Zusammensetzung nach Anspruch 1, worin jeweils jeder aus der Vielzahl von Mikroträgern primär aus einer oder mehr Substanz(en) besteht, die aus der Gruppe ausgewählt sind, bestehend aus Kollagen, Polystyren, Dextran, Polyacrylamid, Cellulose, Calciumalginat, Latex, Polysulfon und Glas.

37. Zusammensetzung nach Anspruch 1, worin die kultivierten Vertebraten-Zellen Fibroblasten darstellen.

38. Zusammensetzung nach Anspruch 1, worin die Mikroträger eine annähernd sphärische Gestalt aufweisen.

39. Zusammensetzung nach Anspruch 37, die weiter Keratinozyten umfasst.

40. Zusammensetzung nach Anspruch 39, worin die Keratinozyten und die Fibroblasten aus dem gleichen Individuum gewonnen werden.

41. Zusammensetzung nach Anspruch 39, die weiter einen oder mehr Keratinozyten-Differenzierungsfaktor(en) umfasst.

42. Zusammensetzung nach Anspruch 41, worin der Keratinozyten-Differenzierungsfaktor Calciumionen in einer Konzentration von 1,5 - 2 mM umfasst.

43. Zusammensetzung nach Anspruch 1, die weiter Endothelzellen umfasst.

44. Zusammensetzung nach Anspruch 37, die weiter Endothelzellen umfasst.

45. Zusammensetzung nach Anspruch 44, worin die Endothelzellen und die Fibroblasten aus dem gleichen Individuum gewonnen werden.

46. Zusammensetzung nach Anspruch 1, worin das Polypeptid aus der Gruppe ausgewählt ist, bestehend aus Interferon-α (IFN-α), Interferon-β (IFN-β), Interferon-γ (IFN-γ), follikelstimulierendem Hormon (FSH), α-Galactosidase, β-Gluceramidase, α-Iduronidase, Iduronat-2-Sulfatase, Glucosamin-N-sulfatase, α-N-Acetylglucosaminidase, Acetyl-Coenzym A:α-Glucosaminid-N-Acetyltransferase, N-Acetylglucosamin-6-sulfatase, β-Galactosidase, N-Acetylgalactosamin-6-sulfatase und β-Glucuronidase.

47. Zusammensetzung nach Anspruch 1, worin das Matrixmaterial zusätzlich eine Substanz umfasst, die aus der Gruppe ausgewählt ist, bestehend aus Heparin, Cellulose, Stärke und Dextran.

48. Zusammensetzung nach Anspruch 1, die weiter Kollagen umfasst.

49. Zusammensetzung nach einem der Ansprüche 1 bis 47 zur Behandlung eines Patienten mit Bedarf an dem Polypeptid.

50. Verfahren zur Herstellung einer Zusammensetzung wie nach Anspruch 1 definiert,
wobei das Verfahren Folgendes umfasst:
Bilden eines Gemischs, umfassend:
(a) eine Vielzahl kultivierter Vertebraten-Zellen, die zur Expression eines Polypeptids genetisch manipuliert sind;
(b) eine Vielzahl von Mikroträgern;
(c) eine Lösung, umfassend lösliches Kollagen; und
(d) ein Mittel, das an ein festes Substrat gebunden ist, worin das Mittel aus der Gruppe ausgewählt ist, bestehend aus einem Faktor, der die Vaskularisation fördert, einem Cytokin, einem Wachstumsfaktor und Ascorbinsäure;
Unterwerfen des löslichen Kollagens im Gemisch den zur Bildung eines Gels wirksamen Bedingungen; und
Exponieren des Gels den Kulturbedingungen, die das Gel zur Kontraktion veranlassen, wodurch der Körper der Zusammensetzung gebildet wird.

51. Verfahren nach Anspruch 50, worin die Lösung eine saure wässrige Lösung aus löslichem Kollagen darstellt und Gelierung durch das Anheben des pH der Lösung herbeigeführt wird.

52. Verfahren nach Anspruch 50, worin der Gelierungsschritt in einer Form stattfindet, so dass das Gel - vor dem Kontraktionsschritt - in der Gestalt der Form vorliegt.

53. Verfahren nach Anspruch 50, worin die kultivierten Vertebraten-Zellen in Gegenwart der Mikroträger, bevor sie mit der Lösung gemischt werden, kultiviert werden.

54. Verfahren nach Anspruch 50, worin das Gel in einer Standform, gefüllt mit dem Gemisch bis zu einer Tiefe von ca. 0,18 mm, gebildet wird.

55. Verfahren nach Anspruch 48, worin das Gel in einer zylindrischen Standform mit einem Innenradius (r) gebildet wird; das Gemisch ein Volumen (V) aufweist; und wenn r in cm ausgedrückt und V in ml ausgedrückt wird, r²/V ca. 1,8 beträgt.

56. Verfahren nach Anspruch 50, weiter umfassend das Zufügen von Keratinozyten zum Körper der Zusammensetzung, nachdem sich das Gel kontrahiert hat.

57. Verwendung einer Population kultivierter Vertebraten-Zellen, die zur Expression eines Polypeptids genetisch manipuliert sind, bei der Herstellung einer Zusammensetzung, wie nach Anspruch 1 definiert, zur Behandlung eines Patienten mit Bedarf daran, worin die kultivierten Vertebraten-Zellen das Polypeptid sezemieren.

58. Verwendung nach Anspruch 57, worin sich die kultivierten Vertebraten-Zellen von einer oder mehr aus einem Patienten entfernten Zelle(n) herleiten und zur Expression und Sekretion des Polypeptids *in vitro* genetisch manipuliert wurden.

59. Verwendung nach Anspruch 57, worin die Zusammensetzung konfiguriert ist, um an einem subkutanen Ort in einen Patienten implantiert zu werden.

60. Verwendung nach Anspruch 57, worin die Zusammensetzung konfiguriert ist, um an einem intraperitonealen, subrenalen-kapsulären, inguinalen, intramuskulären, intraventrikulären, intraomentalen oder intrathekalen Ort in einen Patienten implantiert zu werden.

61. Verwendung nach Anspruch 57, worin die Zusammensetzung zur Behandlung einer Wunde bestimmt ist, worin das Polypeptid eines darstellt, das die Wundheilung fördert und die Zusammensetzung konfiguriert ist, um am Ort einer präexistierenden Wunde eines Patienten implantiert zu werden.

62. Gemisch, das in einer wässrigen Kollagenlösung suspendiert Folgendes umfasst:
(a) eine Vielzahl kultivierter Vertebraten-Zellen, die zur Expression eines Polypeptids genetisch manipuliert sind,
(b) eine Vielzahl von Mikroträgern, und
(c) mindestens ein Mittel, das aus der Gruppe ausgewählt ist, bestehend aus einem Faktor, der die Vaskularisation fördert, einem Cytokin, einem Wachstumsfaktor und Ascorbinsäure, worin das mindestens eine Mittel an ein festes Substrat gebunden ist, das in der Kollagenlösung suspendiert ist,
worin ausreichend Kollagen im Gemisch vorhanden ist, um dem Gemisch zu erlauben, bei einem pH über 5 zu gelieren.

63. Gefäß, enthaltend ein Gemisch, das in einer wässrigen Kollagenlösung Folgendes umfasst:
(a) eine Population kultivierter Vertebraten-Zellen, die zur Expression eines Polypeptids genetisch manipuliert sind;
(b) eine Vielzahl von Mikroträgem; und
(c) ein mit einem festen Substrat assoziiertes Mittel, worin das Mittel aus der Gruppe ausgewählt ist, bestehend aus einem Faktor, der die Vaskularisation fördert, einem Cytokin, einem Wachstumsfaktor und Ascorbinsäure,
worin in dem Gemisch ausreichend Kollagen vorliegt, um dem Gemisch zu erlauben, bei einem pH über 5 zu gelieren.

## Revendications

1. Composition comprenant un corps de matière matricielle comprenant des fibrilles de collagène insolubles,
(a) une population de cellules cultivées de vertébrés manipulées génétiquement pour exprimer un polypeptide;
(b) une pluralité de microporteurs; et
(c) un agent lié à un substrat solide incorporé dans le corps de matière matricielle, où l'agent est sélectionné parmi le groupe consistant en un facteur qui favorise la vascularisation, une cytokine, un facteur de croissance et de l'acide ascorbique,
étant incorporés dans le corps de matière matricielle.

2. La composition de la revendication 1, dans laquelle le substrat solide comprend (a) de l'héparine ou (b) de l'héparane-sulfate protéoglycane.

3. La composition de la revendication 1, dans laquelle le substrat solide comprend de l'agarose avec de l'héparine ou de l'héparane-sulfate protéoglycane lié à celui-ci.

4. La composition de la revendication 3, dans laquelle le substrat solide comprend en outre de l'alginate de calcium.

5. La composition de la revendication 1, dans laquelle le substrat solide comprend en outre de l'alginate de calcium.

6. La composition de la revendication 1, dans laquelle le substrat solide comprend une substance sélectionnée parmi le groupe consistant en collagène, gélatine, acétate d'éthylène-vinyle, copolymère de polylactide/acide glycolique, fibrine, octasulfate de saccharose, dextrane, polyéthylène glycol, un alginate, polyacrylamide, cellulose, latex et méthacrylate de polyhydroxyéthyle.

7. La composition de la revendication 6, dans laquelle de l'héparine ou de l'héparane-sulfate protéoglycane est lié au substrat.

8. La composition de la revendication 1, dans laquelle la composition comprend en outre un deuxième agent sélectionné parmi le groupe consistant en un facteur qui favorise la vascularisation, une cytokine et un facteur de croissance.

9. La composition de la revendication 1, dans laquelle le substrat solide est sous forme de perles.

10. La composition de la revendication 1, dans laquelle le substrat solide est sous forme de fils.

11. La composition de la revendication 1, dans laquelle l'agent est le facteur basique de croissance des fibroblastes (bFGF).

12. La composition de la revendication 1, dans laquelle l'agent est sélectionné parmi le groupe consistant en facteur acide de croissance des fibroblastes (aFGF), en facteur de croissance des cellules endothéliales, en facteur de croissance dérivé des plaquettes (PDGF), en facteur d'angiogenèse stimulant les cellules endothéliales (ESAF), en leucotriène C₄, en prostaglandine, en facteur de croissance type insulinique 1 (IGF-1), en facteur stimulant les colonies de granulocytes (G-CSF), en angiogénine, en facteur transformant de croissance-α (TGF- a), en facteur transformant de croissance-β (TGF- β), en acide ascorbique, en facteur de croissance de l'épiderme (EGF) et en oncostatine M.

13. La composition de la revendication 1, dans laquelle l'agent est sélectionné parmi le groupe consistant en facteur de croissance de l'endothélium vasculaire (VEGF), VEGF-A, VEGF-B, VEGF-C et VEGF-D.

14. La composition de la revendication 1, dans laquelle l'agent est de l'angiopoïétine 1, 2, 3 ou 4.

15. La composition de la revendication 1, comprenant en outre des cellules sécrétant l'agent.

16. La composition de la revendication 1, dans laquelle les cellules cultivées de vertébrés sont sélectionnées parmi le groupe consistant en adipocytes, astrocytes, cellules du muscle cardiaque, chondrocytes, cellules endothéliales, cellules épithéliales, fibroblastes, gangliocytes, cellules glandulaires, cellules gliales, cellules hématopoïétiques, hépatocytes, kératinocytes, myoblastes, cellules neurales, ostéoblastes, celles pancréatiques bêta, cellules rénales, cellules des muscles lisses, cellules des muscles striés et précurseurs de l'une quelconque des cellules ci-dessus.

17. La composition de la revendication 1, dans laquelle les cellules cultivées de vertébrés sont des cellules humaines.

18. La composition de la revendication 1, dans laquelle le polypeptide est sélectionné parmi le groupe consistant en enzymes, hormones, cytokines, facteurs stimulant les colonies, antigènes de vaccins, anticorps, facteurs de coagulation, protéines régulatrices, facteurs de transcription, récepteurs et protéines structurales.

19. La composition de la revendication 1, dans laquelle le polypeptide est le Facteur VIII.

20. La composition de la revendication 1, dans laquelle le polypeptide est une hormone de croissance humaine.

21. La composition de la revendication 1, dans laquelle le polypeptide est le facteur IX.

22. La composition de la revendication 1, dans laquelle le polypeptide est de l'érythropoïétine.

23. La composition de la revendication 1, dans laquelle le polypeptide est sélectionné parmi le groupe consistant en VEGF, VEGF-A, VEGF-B, VEGF-C et VEGF-D.

24. La composition de la revendication 1, dans laquelle le polypeptide est l'insulinotropine.

25. La composition de la revendication 1, dans laquelle le polypeptide est sélectionné parmi le groupe consistant en alpha-1 antitrypsine, calcitonine, glucocérébrosidase, récepteur de lipoprotéines de faible poids moléculaire (LDL), récepteur IL-2, globine, immunoglobuline, anticorps catalytiques, les interleukines, insuline, facteur de croissance type insulinique 1 (IGF-1), hormone parathyroïdienne (PTH), leptine, les facteurs de croissance nerveuse, facteur basique de croissance des fibroblastes (bFGF), FGF acide (aFGF), facteur de croissance de l'épiderme (EGF), facteur de croissance des cellules endothéliales, facteur de croissance dérivé des plaquettes (PDGF), facteurs transformants de croissance, facteur d'angiogenèse stimulant les cellules endothéliales (ESAF), angiogénine, activateur tissulaire du plasminogène (t-PA), facteur stimulant les colonies de granulocytes ( G-CSF) et le facteur stimulant les colonies de granulocytes-macrophages (GM-CSF).

26. La composition de la revendication 1, dans laquelle les microporteurs sont des perles de collagène de type I.

27. La composition de la revendication 26, dans laquelle les microporteurs sont poreux.

28. La composition de la revendication 1, dans laquelle les microporteurs sont des perles de gélatine poreuse.

29. La composition de la revendication 1, dans laquelle la majorité des microporteurs a une forme approximativement sphérique et a un diamètre d'entre approximativement 0,1 et approximativement 2mm.

30. La composition de la revendication 1, dans laquelle la matière matricielle comprend en plus une substance sélectionnée parmi le groupe consistant en un deuxième type de collagène, en agarose, en alginate, en fibronectine, en laminine, en acide hyaluronique, en héparane-sulfate, en dermatane-sulfate, en protéoglycanes sulfatés, en fibrine, en élastine et en tenascine.

31. La composition de la revendication 1, comprenant en plus des fibres non de collagène dispersées dans le corps de la matière matricielle.

32. La composition de la revendication 31, dans laquelle les fibres non de collagène comprennent une matière sélectionnée parmi le groupe consistant en nylon, dacron, polytétrafluoro-éthylène, acide polyglycolique, copolymère d'acide polylactique/polyglycolique, polystyrène, chlorure de polyvinyle, boyau, coton, lin, polyester et soie.

33. La composition de la revendication 1, configurée pour être implantée chez un patient.

34. La composition de la revendication 33, dans laquelle les cellules cultivées de vertébrés sont dérivées d'une ou de plusieurs cellules prélevées du patient.

35. La composition de la revendication 34, dans laquelle les cellules cultivées de vertébrés consistent en une population clonale.

36. La composition de la revendication 1, dans laquelle chacun d'une pluralité de microporteurs consiste essentiellement en une ou plusieurs subtances sélectionnées parmi le groupe consistant en collagène, polystyrène, dextrane, polyacrylamide, cellulose, alginate de calcium, latex, polysulfone et verre.

37. La composition de la revendication 1, dans laquelle les cellules cultivées de vertébrés sont des fibroblastes.

38. La composition de la revendication 1, dans laquelle les microporteurs ont une forme approximativement sphérique.

39. La composition de la revendication 37, comprenant en outre des kératinocytes.

40. La composition de la revendication 39, dans laquelle les kératinocytes et les fibroblastes sont obtenus du même individu.

41. La composition de la revendication 39, comprenant en outre un ou plusieurs facteurs de différenciation des kératinocytes.

42. La composition de la revendication 41, dans laquelle le facteur de différenciation des kératinocytes comprend des ions de calcium à une concentration de 1,5-2 mM.

43. La composition de la revendication 1, comprenant en outre des cellules endothéliales.

44. La composition de la revendication 37, comprenant en outre des cellules endothéliales.

45. La composition de la revendication 44, dans laquelle les cellules endothéliales et les fibroblastes sont obtenus du même individu.

46. La composition de la revendication 1, dans laquelle le polypeptide est sélectionné parmi le groupe consistant en interféron-α (IFN- α), interféron-β (IFN- β), interféron-γ (IFN- γ), hormone stimulant les follicules (FSH), α-galactosidase, β-glucéramidase, α-iduronidase, iduronate-2-sulfatase, glucosamine-N-sulfatase, α-N-acétyl-glucosaminidase, acétyl-coenzyme A: α-glucosaminide-N-acétyltransférase, N-acétylglucosamine-6-sulfatase, β-galactosidase, N-acétylgalactosamine-6-sulfatase et β-glucuronidase.

47. La composition de la revendication 1, dans laquelle la matière matricielle comprend en plus une substance sélectionnée parmi le groupe consistant en héparine, cellulose, amidon et dextrane.

48. La composition de la revendication 1, comprenant en outre du collagène.

49. Composition selon l'une quelconque des revendications 1 à 47, pour le traitement d'un patient ayant besoin du polypeptide.

50. Procédé de fabrication d'une composition telle que définie dans la revendication 1, le procédé comprenant
former un mélange comprenant:
(a) une pluralité de cellules cultivées de vertébrés manipulées génétiquement pour exprimer un polypeptide;
(b) une pluralité de microporteurs;
(c) une solution comprenant du collagène soluble; et
(d) un agent lié à un substrat solide, où l'agent est sélectionné parmi le groupe consistant en un facteur qui favorise la vascularisation, une cytokine, un facteur de croissance et de l'acide ascorbique;
soumettre le collagène soluble dans le mélange à des conditions efficaces pour former un gel; et
exposer le gel à des conditions de culture qui causent la contraction du gel, formant ainsi le corps de la composition.

51. Le procédé de la revendication 50, dans lequel la solution est une solution aqueuse acide de collagène soluble, et la gélification est réalisée en élevant le pH de la solution.

52. Le procédé de la revendication 50, dans lequel l'étape de gélification a lieu dans un moule de sorte que, préalablement à l'étape de contraction, le gel a la forme du moule.

53. Le procédé de la revendication 50, dans lequel les cellules cultivées de vertébrés sont cultivées en présence des microporteurs préalablement à leur mélange avec la solution.

54. Le procédé de la revendication 50, dans lequel le gel est formé dans un moule à fond plat rempli avec le mélange à une profondeur d'environ 0,18 mm.

55. Le procédé de la revendication 48, dans lequel le gel est formé dans un moule cylindrique à fond plat ayant un rayon interne (r); le mélange a un volume (V); et lorsque r est exprimé en cm et V est exprimé en ml, r²/V fait environ 1,8.

56. Le procédé de la revendication 50, comprenant en outre ajouter des kératinocytes au corps de la composition après la contraction du gel.

57. L'utilisation d'une population de cellules cultivées de vertébrés manipulées génétiquement pour exprimer un polypeptide, dans la préparation d'une composition telle que définie dans la revendication 1 pour le traitement d'un patient ayant besoin de celle-ci, où les cellules cultivées de vertébrés sécrètent le polypeptide.

58. L'utilisation de la revendication 57, dans laquelle les cellules cultivées de vertébrés sont dérivées d'une ou de plusieurs cellules prélevées d'un patient, et ont été manipulées génétiquement *in vitro* pour exprimer et sécréter le polypeptide.

59. L'utilisation de la revendication 57, dans laquelle la composition est configurée pour être implantée à un site sous-cutané chez un patient.

60. L'utilisation de la revendication 57, dans laquelle la composition est configurée pour être implantée à un site intrapéritonéal, sous la capsule rénale, intramusculaire, intraventriculaire, intra-épiploïque ou intrarachidien chez un patient.

61. L'utilisation de la revendication 57, dans laquelle la composition est destinée au traitement d'une plaie, où le polypeptide en est un qui favorise la guérison des plaies, et la composition est configurée pour être implantée au site d'une plaie pré-existante chez un patient.

62. Mélange comprenant, en suspension dans une solution aqueuse de collagène,
(a) une pluralité de cellules cultivées de vertébrés manipulées génétiquement pour exprimer un polypeptide;
(b) une pluralité de microporteurs; et
(c) au moins un agent sélectionné parmi le groupe consistant en un facteur qui favorise la vascularisation, une cytokine, un facteur de croissance et de l'acide ascorbique, où le au moins un agent est lié à un substrat solide en suspension dans la solution de collagène,
où il y a suffisamment de collagène dans le mélange pour permettre au mélange de se gélifier à un pH supérieur à 5.

63. Récipient comprenant un mélange comprenant, en suspension dans une solution aqueuse de collagène,
(a) une population de cellules cultivées de vertébrés manipulées génétiquement pour exprimer un polypeptide;
(b) une pluralité de microporteurs; et
(c) un agent associé à un substrat solide, où l'agent est sélectionné parmi le groupe consistant en un facteur qui favorise la vascularisation, une cytokine, un facteur de croissance et de l'acide ascorbique,
où il y a suffisamment de collagène dans le mélange pour permettre au mélange de se gélifier à un pH supérieur à 5.
